# EUROPEAN PATENT SPECIFICATION

(11) **EP 4 269 614 B1**
(45) Date of publication and mention of the grant of the patent: **13.05.2026**
(21) Application number: 21910876.8
(22) Date of filing: 22.12.2021
(51) Int. Cl.: C12Q 1/686, C12Q 1/6851

(54) **METHOD FOR DETECTING COPY NUMBER OF SPECIFIC NUCLEIC ACID PER SINGLE CELL**
VERFAHREN ZUM NACHWEIS DER KOPIENZAHL EINER SPEZIFISCHEN NUKLEINSÄURE PRO EINZELZELLE
PROCÉDÉ DE DÉTECTION DU NOMBRE DE COPIES D'UN ACIDE NUCLÉIQUE SPÉCIFIQUE PAR CELLULE UNIQUE

(30) Priority: 25.12.2020 JP 2020216149
(43) Date of publication of application: 01.11.2023
(73) Proprietor: TL Genomics Inc., Koganei-shi, Tokyo 184-0012 (JP)
(72) Inventor: TAKAHASHI, Keita, Koganei-shi, Tokyo 184-0012 (JP); KUBO, Tomohiro, Koganei-shi, Tokyo 184-0012 (JP); TSUCHIYA, Junichi, Koganei-shi, Tokyo 184-0012 (JP)
(74) Representative: Papula Oy
(86) International application number: PCT/JP2021/047619
(87) International publication number: WO 2022/138736

(56) References cited:
- WO-A1-2018/025856
- WO-A1-2018/123220
- WO-A1-2021/200749
- JP-A- 2015 508 655
- JP-A- 2015 533 079
- JP-A- 2020 000 011
- US-A1- 2014 134 613
- CHANG CHIA-HAO ET AL: "Evaluation of digital real-time PCR assay as a molecular diagnostic tool for single-cell analysis", vol. 8, no. 1, 21 February 2018 (2018-02-21), US, XP093271143, ISSN: 2045-2322, Retrieved from the Internet <URL:https://www.nature.com/articles/s41598-018-21041-5.pdf> DOI: 10.1038/s41598-018-21041-5
- YING ZHU ET AL: "Printing 2-Dimentional Droplet Array for Single-Cell Reverse Transcription Quantitative PCR Assay with a Microfluidic Robot", SCIENTIFIC REPORTS, vol. 5, no. 1, 1 April 2015 (2015-04-01), XP055743284, DOI: 10.1038/srep09551
- TIAN YU ET AL: "Microfluidics-based digital quantitative PCR for single-cell small RNA quantification+", BIOLOGY OF REPRODUCTION, vol. 97, no. 3, 31 August 2017 (2017-08-31), US, pages 490 - 496, XP055591868, ISSN: 0006-3363, DOI: 10.1093/biolre/iox102
- TAN CHIANRU ET AL: "A multiplex droplet digital PCR assay for non-invasive prenatal testing of fetal aneuploidies", ANALYST, vol. 144, no. 7, 8 January 2019 (2019-01-08), UK, pages 2239 - 2247, XP055820640, ISSN: 0003-2654, DOI: 10.1039/C8AN02018C
- SATO TAISUKE, ITO YUKI, SAMURA OSAMU, AOKI HIROAKI, UCHIYAMA TORU, OKAMOTO AIKOU, HATA KENICHIRO: "Direct Assessment of Single-Cell DNA Using Crudely Purified Live Cells: A Proof of Concept for Noninvasive Prenatal Definitive Diagnosis", THE JOURNAL OF MOLECULAR DIAGNOSTICS, vol. 22, no. 2, 1 February 2020 (2020-02-01), pages 132 - 140, XP009536932, ISSN: 1525-1578, DOI: 10.1016/j.jmoldx.2019.10.006

## Description

### Technical Field

The present invention relates to a method for detecting the copy number of a specific nucleic acid per single cell.

### Background Art

Non Patent Literature (hereinafter, referred to as NPL) 1 discloses encapsulating genomic DNA extracted from cells in droplets and amplifying the genomic DNA by Polymerase Chain Reaction (PCR), although NPL 1 is not directed to a single-cell analysis. In NPL 1, a target sample and a reference sample are independently quantified in the same droplet. This analysis is achieved by distinguishing between the fluorescence signals of intercalators to distinguish between the lengths of amplicons.

NPL 2 discloses single-cell RT-PCR (reverse transcription PCR) targeting mRNA of a specific gene. In NPL 3, the presence of HIV-1 in CD4⁺ T cells is detected with high throughput by a reverse transcription reaction and a single-cell-in-droplet (scd) PCR assay.

NPL 4 discloses a single cell-based droplet digital PCR (sc-ddPCR) method. In this method, single cells are encapsulated in droplets and PCR is performed within the droplets using gene-specific primers and probes. One copy of the gene is artificially introduced into a cell.

NPL 5 discloses lysing a single cell within a droplet and combining the droplet with the single cell lysed therein with the droplet of the RT-PCR reaction solution.

Patent Literature (hereinafter, referred to as PTL) 1 discloses analyzing, although not single cell analysis, the characteristics of DNA obtained from enriched fetal cells from maternal blood to determine its genetic status. PTL 1 discloses amplifying the DNA of the enriched fetal cells and analyzing the amplified DNA by using digital PCR. In addition, PTL 1 discloses that chromosome 21 is detected.

PTL 2 discloses that blood cells potentially containing fetal cells are each isolated at the single cell level, and chromosomal DNA is independently extracted from the isolated blood cells, and fractions containing the fetal-derived chromosomal DNA are identified in an after-the-fact manner.

NPL 6 discloses detecting an SRY gene on genomic DNA of fetal cells circulating in maternal blood by the above-described sc-ddPCR method.

NPL 7 discloses quantitative PCR using 1, 2, 4 ... copies of a yeast genome weighed by using the piezoelectric effect as a template.

NPLs 8 and 9 will be described below. NPL 10 discloses digital quantitative PCR "dqPCR" for copy number detections in single cells. NPL 11 discloses multiplex digital PCR methods using probes of different wavelengths.

### Citation List

### Patent Literature

PTL 1
   Japanese Unexamined Patent Application Publication (Translation of PCT Application) No. 2014-533509
PTL 2
   Japanese Patent Application Laid-Open No. 2018-102242

### Non Patent Literature

NPL 1
   Laura Miotke, et al., "High Sensitivity Detection and Quantitation of DNA Copy Number and Single Nucleotide Variants with Single Color Droplet Digital PCR", Analytical chemistry, 86, 5, 2618-2624.
NPL 2
   Dennis J. Eastburn, et al., "Identification and genetic analysis of cancer cells with PCR-activated cell sorting", Nucleic Acids Research, 42, 16, e128.
NPL 3
   Robert W. Yucha, et al., "High-throughput Characterization of HIV-1 Reservoir Reactivation Using a Single-Cell-in-Droplet PCR Assay", EBioMedicine, 20, 217-229.
NPL 4
   Yuka Igarashi, et al., "Single Cell-Based Vector Tracing in Patients with ADA-SCID Treated with Stem Cell Gene Therapy", Molecular Therapy, Methods & Clinical Development, 6, 8-16.
NPL 5
   Samuel C. Kim, et al., "Single-Cell RT-PCR in Microfluidic Droplets with Integrated Chemical Lysis", Analytical Chemistry, 90, 2, 1273-1279.
NPL 6
   Taisuke Sato, et al., "Direct Assessment of Single-Cell DNA Using Crudely Purified Live Cells: A Proof of Concept for Noninvasive Prenatal Definitive Diagnosis", The Journal of Molecular Diagnostics, 22, 2, 2, 132-140.
NPL 7
   Unoh Ki, et al., "A Novel Bioprinting Application for the Production of Reference Material Containing a Defined Copy Number of Target DNA", Ricoh Technical Report, 2020, 44, 14-26. Retrieved from <https://jp.ricoh.com/technology/techreport/44/>
NPL 8
   Ichiro Hanamura, et al., "Frequent gain of chromosome band 1q21 in plasma-cell dyscrasias detected by fluorescence in situ hybridization: incidence increases from MGUS to relapsed myeloma and is related to prognosis and disease progression following tandem stem-cell transplantation", Blood, 108, 5, 1724-1732.
NPL 9
   ISHIDA Tadao, "Tahatusei-kotujuishu: Senshokutai ijou to Rinshou byougata·Yogo (Multiple myeloma: chromosomal abnormalities, clinical forms, and prognosis)," [online], 2013-09-21, Clinical Hematology, 54, 10, 2, 1856-1866, Retrieved from https://doi.org/10.11406/rinketsu.54.1856
NPL 10
   Chang, Chia-Hao, et al. "Evaluation of digital real-time PCR assay as a molecular diagnostic tool for single-cell analysis", Scientific reports, 8, 1, 3432.
NPL 11
   Tan, Chianru, et al. "A multiplex droplet digital PCR assay for non-invasive prenatal testing of fetal aneuploidies", Analyst, 144, 7, 2239-2247.

### Summary of Invention

### Technical Problem

Digital PCR qualitatively indicates the presence or absence of a template within a minute compartment (for example, droplet). Therefore, digital PCR is not suitable for detecting a discrete copy number variation (CNV) for a specific nucleic acid within a minute compartment.

An object of the present invention is to provide a method capable of detecting the copy number of a specific nucleic acid per single cell in a population of cells (herein also referred to as "cell population").

### Solution to Problem

The present invention relates to a method for detecting the copy number of a specific nucleic acid per single cell as described below.
[1] A method for detecting a copy number of a specific nucleic acid per single cell in a cell population, the method comprising:
   amplifying, in a reaction compartment of a plurality of reaction compartments containing a Polymerase Chain Reaction (PCR) system and a DNA sample that is derived from a nucleic acid in a single cell, a target contained in the DNA sample by PCR; and
   quantifying an amplicon obtained by the PCR in each of the plurality of reaction compartments during an exponential amplification phase.
[2] The method according to [1, wherein the DNA sample is a set of genomic DNA in the single cell, a reverse transcription product of RNA in the single cell, or mitochondrial DNA in the single cell.
[3] The method according to [1] or [2], the amplicon is quantified by a fluorescent probe method.
[4] The method according to [3], wherein:
   the PCR reaction system contains a plurality of probes including fluorescent materials with fluorescence wavelengths different from each other, respectively, the plurality of probes being respectively assigned to regions different from each other on the DNA sample;
   each of the regions contains the target or a plurality of the targets; and
   in the quantifying the amplicon, by detecting fluorescence of all of a plurality of wavelengths from the reaction compartment, the reaction compartment in which the target or the plurality of targets contained in the DNA sample are amplified is distinguished from the reaction compartment in which only a contaminating cell-free nucleic acid is amplified.
[5] The method according to [3] or [4], wherein:
   each of the regions contains a plurality of targets to which a plurality of probes including fluorescent materials with fluorescence wavelengths identical to each other are assigned, respectively; and
   in the quantifying the amplicon, for each of the regions, the amplicon is quantified by collectively measuring fluorescence from the plurality of probes, regardless of a difference between the targets.
[6] The method according to [3], wherein:
   the DNA sample contains a set of genomic DNA in the single cell and mitochondrial DNA in the single cell;
   the PCR reaction system contains a first probe including a fluorescent material with a first fluorescence wavelength and a second probe including a fluorescent material with a second fluorescence wavelength, the first probe being assigned to a region on the genomic DNA, the second probe being assigned to the mitochondrial DNA;
   the region includes the target or a plurality of the targets;
   in the amplifying by the PCR, in each of the plurality of reaction compartments, the target or the plurality of targets contained in the set of genomic DNA and a target contained in the mitochondrial DNA are amplified by the PCR;
   in the quantifying the amplicon, the amplicon of the genomic DNA and the amplicon of mitochondrial DNA are quantified in each of the plurality of reaction compartments in a cycle during which amplification of the target contained in the mitochondrial DNA reaches a plateau and amplification of the target or the plurality of targets contained in the genomic DNA reaches the exponential amplification phase; and
   in the quantifying the amplicon, by detecting fluorescence of both the first fluorescence wavelength and the second fluorescence wavelength from the reaction compartment, the reaction compartment in which the target or the plurality of targets contained in the genomic DNA and the target contained in the mitochondrial DNA are amplified is distinguished from the reaction compartment in which only a contaminating cell-free nucleic acid is amplified.
[7] The method according to any one of [1] to [6], further comprising, generating the reaction compartment by lysing the single cell in a compartment containing the single cell, a cell lysis reagent, and a PCR premix.
[8] The method according to any one of [1] to [6], further comprising:
   lysing the single cell in a compartment containing the single cell; and
   generating the reaction compartment by combining the compartment containing the single cell lysed with a compartment containing a PCR premix.
[9] The method according to any one of [1] to [6], further comprising:
   mixing a population of cell nuclei and a PCR premix in bulk; and
   generating a plurality of the reaction compartments by separating the cell nuclei in the population of the cell nuclei together with the PCR premix from each other.
[10] The method according to any one of [1] to [9], wherein the reaction compartment is a reaction droplet that is a droplet containing the DNA sample and the PCR reaction system.
[11] The method according to any one of [1] to [10], wherein:
   the DNA sample contains a set of genomic DNA in the single cell; and
   the method further comprises detecting, from a result of the quantifying the amplicon, a presence of the single cell with at least one chromosomal mutation selected from the group consisting of aneuploidy over an entire length of a chromosome, partial aneuploidy of a chromosome, gene amplification, and gene deletion.
[12] The method according to [11], wherein the single cell with the at least one chromosomal mutation contains the genomic DNA at least with a chromosomal mutation selected from below:
   - aneuploidy of chromosome 21,
   - aneuploidy of chromosome 18,
   - aneuploidy of chromosome 13,
   - aneuploidy of Y chromosome,
   - aneuploidy of X chromosome,
   - deletion of the 22q11.2 region on a long arm of chromosome 22,
   - deletion of the 5q region on a short arm of chromosome 5,
   - deletion of the 15q11-q13 region on a long arm of chromosome 15,
   - amplification of a long arm of chromosome 1,
   - deletion of a short arm of chromosome 17,
   - deletion of a long arm of chromosome 13,
   - deletion of a long arm of chromosome 4,
   - deletion of a long arm of chromosome 5,
   - deletion of a long arm of chromosome 7,
   - amplification of chromosome 8,
   - deletion of chromosome 11,
   - aneuploidy of chromosome 12,
   - deletion of a long arm of chromosome 20,
   - deletion of a long arm of chromosome 19,
   - deletion of chromosome 1,
   - deletion of a long arm of chromosome 18,
   - deletion of a short arm of chromosome 8,
   - deletion of chromosome 4,
   - amplification of a long arm of chromosome 8,
   - deletion of a long arm of chromosome 16,
   - amplification of a short arm of chromosome 5,
   - amplification of a long arm of chromosome 3,
   - deletion of a short arm of chromosome 3,
   - deletion of a short arm of chromosome 9,
   - gene amplification of MYCN gene,
   - gene amplification of HER2 gene, and
   - gene amplification of MET gene.
[13] The method according to [11], further comprising:
   generating data that includes information on whether or not the single cell with the at least one chromosomal mutation is detected, wherein
   the cell population is isolated from amniotic fluid or maternal blood so as to contain a fetal cell; and
   the data is provided for a diagnosis of trisomy 13, trisomy 18, trisomy 21, Turner syndrome, triple X syndrome, XYY syndrome, Klinefelter syndrome, Di George syndrome, Angelman syndrome, Prader-Willi syndrome, or cri-du-chat syndrome.
[14] The method according to [11], further comprising:
   generating data that includes information on whether or not the single cell with the at least one chromosomal mutation is detected, wherein
   the cell population is isolated from a patient, and
   the data is provided for a diagnosis of myelodysplastic syndrome, multiple myeloma, idiopathic eosinophilia, chronic eosinophilic leukemia, acute nonlymphocytic leukemia, myeloproliferative neoplasm, chronic lymphocytic leukemia, acute myeloid leukemia, brain tumor, neuroblastoma, colon cancer, breast cancer, ovarian cancer, cervical cancer, endometrial cancer, small cell lung cancer, non-small cell lung cancer, bladder cancer, kidney cancer, liver cancer, pancreatic cancer, esophageal cancer, thyroid cancer, or head and neck cancer.

### Advantageous Effects of Invention

The present invention can detect the copy number of a specific nucleic acid per single cell in a cell population. For example, the present invention can detect a copy number variation (CNV) of a specific nucleic acid per single cell.

### Brief Description of Drawings

FIG. 1 is a conceptual diagram of detection;
FIG. 2 illustrates a flowchart illustrating an exemplary detection;
FIG. 3 illustrates an overview of droplet preparation;
FIG. 4 illustrates the assignment of a probe;
FIG. 5 illustrates a scatter of detection events;
FIG. 6 illustrates the assignment of a plurality of probes;
FIG. 7 illustrates a scatter of detection events with enhanced fluorescence;
FIG. 8 illustrates scatters of droplets in a plateau;
FIG. 9 illustrates scatters of droplets during an exponential amplification phase;
FIG. 10 illustrates scatters of droplets with enhanced fluorescence;
FIG. 11 illustrates scatters of droplets in heterogeneous cell populations;
FIG. 12 illustrates scatters of droplets in heterogeneous cell populations;
FIG. 13 illustrates scatters distinguishing between diploidy and monoploidy;
FIG. 14 illustrates scatters distinguishing between diploidy and monoploidy;
FIG. 15 illustrates scatters distinguishing between diploidy and monoploidy;
FIG. 16 illustrates scatters distinguishing between diploidy and monoploidy with enhanced fluorescence;
FIG. 17 illustrates scatters distinguishing between diploidy and monoploidy with enhanced fluorescence;
FIG. 18 illustrates scatters distinguishing between diploidy and monoploidy with enhanced fluorescence;
FIG. 19 illustrates boxplots corresponding to respective scatters;
FIG. 20 illustrates a ROC curve when using 23 types of probes;
FIG. 21 illustrates a scatter with a straight line indicating a cutoff value;
FIG. 22 illustrates cluster displacement for each measurement;
FIG. 23 illustrates a graph showing variations in corrected measured values;
FIG. 24 is a diagram for explaining calculation of a cutoff value using corrected measured values;
FIG. 25 is a scatter of droplets containing genomic DNA derived from human blood;
FIG. 26 illustrates a scatter of droplets containing CD45 cells;
FIG. 27 illustrates scatters of droplets in homozygous cell populations;
FIG. 28 illustrates scatters of droplets in heterogeneous cell populations;
FIG. 29 illustrates scatters of droplets for respective cell populations;
FIG. 30 illustrates a boxplot showing the detection results of abnormal droplets for respective cell populations;
FIG. 31 illustrates a scatter of droplets containing RNA reverse transcription product;
   and
FIG. 32 illustrates a scatter of droplets containing mitochondrial DNA.

### Description of Embodiments

### Overview

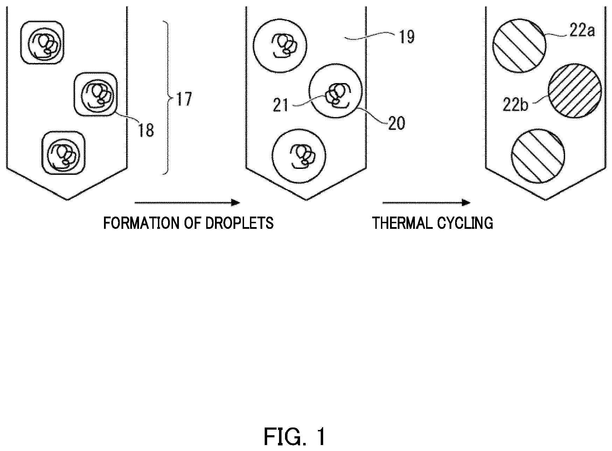

FIG. 1 conceptually illustrates the detection of the copy number of a specific nucleic acid per single cell in a cell population, for example, to detect a CNV at a specific position on genomic DNA. In one aspect, the cells are human cells.

Hereinafter, in describing the CNV, the term "monoploid (monoploidy)" includes haploidization (i.e., monosomy) of all of a specific chromosome, as well as partially monoploid states due to deletions. The CNV includes all or part of a diploid chromosome becoming monoploid.

The term "triploid (triploidy)" includes triploidization (i.e., trisomy) of all of a specific chromosome, as well as partially triploid states due to duplication. The CNV includes all or part of a diploid chromosome becoming triploid. The CNV includes all or part of a diploid chromosome becoming to have the ploidy greater than that of triploid.

The CNV also includes ploidy changes due to duplication of all or part of a sex chromosome in a male, which is normally monoploid. In the following, monoploid, diploid, triploid and other polyploid are referred to as "polyploidy" in some cases.

The CNV also includes changes resulting in the complete loss of all or part of a polyploid chromosome (biallelic deletion). Further, the CNV also includes changes that result in all or part of a chromosome that is not normally present.

In the present invention, detection of cells with aneuploid chromosomes includes the following: detecting that a cell population containing cells with euploid chromosomes also contains cells with aneuploid chromosomes. In the present invention, detection of cells with aneuploid chromosomes includes the following: detecting that a cell population containing cells with euploid chromosomes is a cell population that did not contain cells with aneuploid chromosomes, or contains aneuploid cells below the limit of detection. In the present invention, detection of cells with aneuploid chromosomes includes the following: detecting that a cell population contains cells with aneuploid chromosomes but does not contain cells with euploid chromosomes.

### Overall flow

As illustrated in FIG. 1 on the left side, cell population 17 is provided. Cell population 17 is a collection of single cells 18. The cells may be eukaryotic or prokaryotic. In one aspect, the cell is a eukaryotic cell with one nucleus.

As illustrated in FIG. 1 in the center, reaction compartments each containing DNA sample derived from a nucleic acid in single cell 18 and a PCR reaction system are prepared. In FIG. 1, droplets 20 are formed in oil 19 as reaction compartments. The drawing shows one aspect of the droplet. Droplet 20 is a water-in-oil emulsion type droplet. In one aspect, the volume of the reaction compartment (droplet 20) is from 1 pL (picoliter) to 1 µL (microliter) or from 1 pL (picoliter) to 10 nL (nanoliter). In this aspect, the volume of the reaction compartment (droplet 20) is 10 pL, 100 pL, 1 nL, 10 nL, or 100 nL. The reaction compartment may have any configuration as long as the compartment is a minute compartment containing DNA sample and a PCR reaction system, and may be, for example, a microwell.

The DNA sample is, for example, a set of genomic DNA in a single cell, a reverse transcription product of RNA in a single cell, or DNA of a mitochondrial genome in a single cell (hereinafter referred to as "mitochondrial DNA"). In FIG. 1, droplet 20 contains a set of genomic DNA 21 in single cell 18 as DNA sample. Droplet 20 further contains a PCR reaction system. Hereinafter, droplet 20 may be referred to as a reaction droplet.

In one aspect, the DNA sample is a set of genomic DNA in a single cell. A "set of genomic DNA" may be interpreted as genomic DNA as it is extracted. In one aspect, no whole genome amplification is performed on the set of genomic DNA. Allele dropout to be caused by the whole genome amplification can be thus avoided. The "set of genomic DNA" includes those unintentionally lost in part during cell lysis or other extraction procedures. In one aspect, the "set of genomic DNA" does not include mitochondrial DNA. However, for using a set of genomic DNA as the DNA sample, the reaction compartment may be contaminated with mitochondrial DNA.

In one aspect, the DNA sample is the reverse transcription product of RNA within a single cell.

In one aspect, the DNA sample is mitochondrial DNA in a single cell.

In one aspect, the DNA sample is a set of genomic DNA in a single cell and mitochondrial DNA in the same single cell. In this case, mitochondrial DNA may be used as a control for detecting the copy number of chromosomal genomic DNA.

In another aspect, the DNA sample is the nucleic acid of a microorganism or virus that has invaded a cell, or a nucleic acid that has been introduced into a cell.

As illustrated in FIG. 1 on the right side, the reaction compartment is subject to thermal cycling. That is, in the reaction compartment containing the PCR reaction system and the DNA sample derived from the nucleic acid in the single cell, the target contained in the DNA sample is amplified by PCR. In FIG. 1, a target contained in the set of genomic DNA 21 is amplified by PCR within droplet 20. In PCR, DNA sample (for example, genomic DNA) serves as a template. In FIG. 1, droplets 22a and 22b are reaction droplets during the exponential amplification phase. At this stage, the amplicons are quantified for respective reaction droplets. That is, PCR amplicons (amplicons obtained by the PCR) are quantified in each reaction compartment (for example, reaction droplet) during the exponential amplification phase. In one aspect, the quantification of the amplicons is performed by a fluorescent probe method.

In FIG. 1, droplet 22b exhibits a stronger signal than droplet 22a. Therefore, the copy number of the genomic region of the subject of the single cell corresponding to droplet 22b is greater than the copy number of the genomic region of the subject of the single cell corresponding to droplet 22a. Therefore, the copy number and CNV of genomic DNA for a specific location (target location) on the genome can be detected.

### Flow of detection

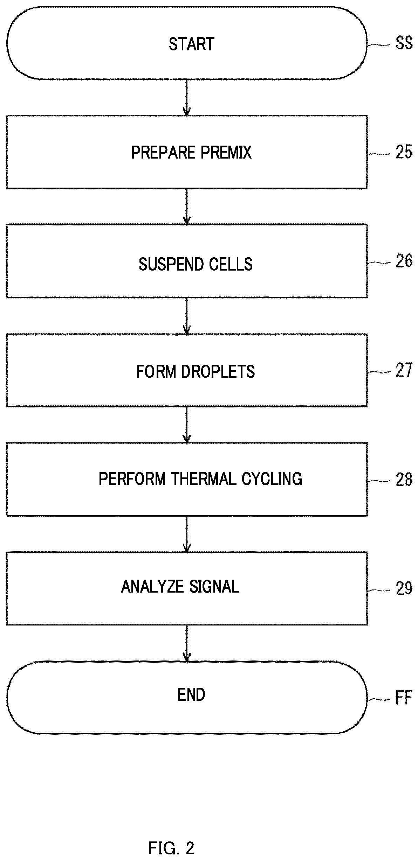

FIG. 2 is a flowchart illustrating exemplary detection of the copy number of a nucleic acid. In the flowchart, an example using a droplet as a reaction compartment and a set of genomic DNA in a single cell as DNA sample will be described.

In step 25 after the start, a PCR premix is prepared. The premix contains a primer, a DNA polymerase, a deoxynucleoside triphosphate (dNTP), a buffer and necessary cofactors. In one aspect, the cofactors include magnesium ions. In one aspect, a cell lysis reagent is mixed with the premix in advance. Examples of the cell lysis reagent include sodium dodecyl sulfate, TRITON X-100 (TRITON is a registered trademark), and NP-40 (trademark). The premix includes necessary probes.

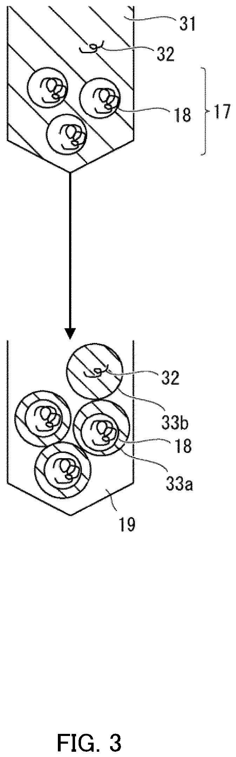

In step 26 illustrated in FIG. 2, cell population 17 is suspended in premix 31, as illustrated in FIG. 3 on the upper side. The step after the suspension until droplet formation is carried out quickly so that the cell lysis reagent and cells do not react with each other.

In step 27 illustrated in FIG. 2, individual single cell 18 together with premix 31 is formed into a droplet, as illustrated in FIG. 3 on the lower side. As a result, droplet 33a containing single cell 18 is obtained.

QX200 Droplet Generator (Bio-Rad Laboratories, Inc.), for example, can be used as a system for forming a single cell together with the premix into a droplet. The system for forming droplets is not limited thereto.

In one aspect illustrated in FIG. 3, a cell lysis reagent is added to droplet 33a. In one aspect, single cell 18 in droplet 33a is lysed within droplet 33a with the above-described cell lysis reagent. A reaction droplets is obtained by the cell lysis. That is, the reaction compartment (reaction droplet) is generated by lysing single cell 18 in the compartment (droplet 33a) containing single cell 18, a cell lysis reagent, and a PCR premix. Reaction of the cell lysis reagent proceeds efficiently by heating.

In another aspect of step 27 illustrated in FIG. 2, the single cell is lysed in a compartment (droplet) containing the single cell before mixing with the premix. Further, the compartment (droplet) containing the lysed single cell is combined with the compartment (droplet) containing the PCR premix. A reaction compartment (reaction droplet) can be obtained by the combining.

In another aspect of step 27 illustrated in FIG. 2, before mixing the single cells with the premix, the bulk cells are lysed to obtain a population of bulk cell nuclei. The population of bulk cell nuclei is mixed with the PCR premix. A reaction compartments (reaction droplets) are generated by separating the cell nuclei in the population of the cell nuclei together with the premix from each other.

In step 28 illustrated in FIG. 2, PCR proceeds in the reaction droplet by thermal cycling. The thermal cycling stops at the exponential amplification phase of PCR. In one aspect, the "exponential amplification phase" refers to a state where template-dependent DNA synthesis is not restricted because dNTPs, primer sets, probe sets and active enzymes are not depleted. In one aspect, the "exponential amplification phase" refers to a state where the chain reaction has not reached a plateau.

In step 29 illustrated in FIG. 2, the signal from the reaction compartment (reaction droplet) is analyzed to quantify the copy number of the target in the reaction droplet. The processing then ends.

In one aspect of step 25 illustrated in FIG. 2, the premix further includes fluorescent probes that hybridize to the target or amplicons obtained from the target. In another aspect, the premix further contains a fluorescent intercalator that binds to DNA.

In one aspect, the number of targets of the probes contained within the amplicon is one. In another aspect, the number of targets of the probes contained within the amplicon is two or more. As the plurality of probes, which bind to one amplicon, respectively include fluorescent materials that emit fluorescence of the same color, the fluorescence emitted from the amplicon can be enhanced.

### Two-color probe method

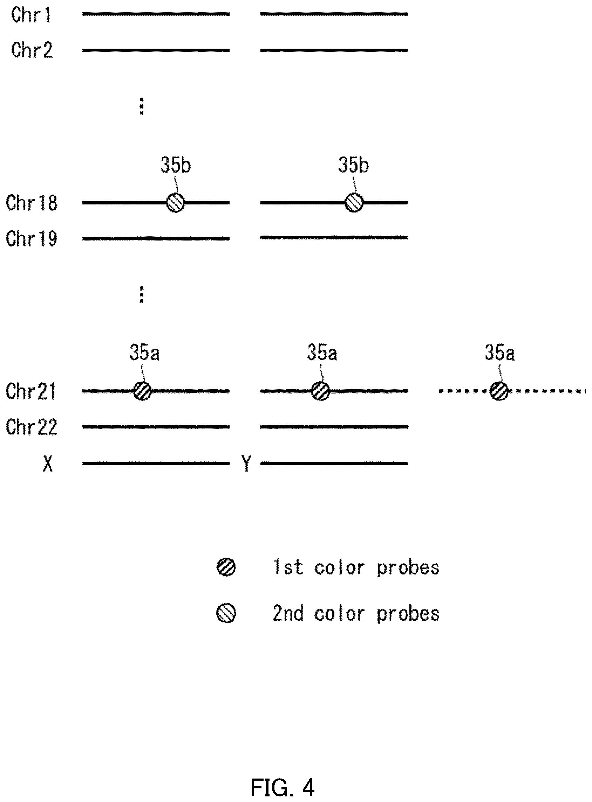

FIG. 4 shows an example of the assignment of two color probes. Probe 35a (1st color) including a fluorescent material with a first fluorescence wavelength is assigned to a target on chromosome 21. Probe 35b (2nd color) including a fluorescent material with a second fluorescence wavelength is assigned to a target on chromosome 18. These chromosome types are exemplary only. These probes are mixed together in the reaction compartment (reaction droplet).

In this example, the target to which the probe of the first fluorescence wavelength is assigned, or a region containing the target, is used as a subject for copy number (CNV) detection. In this example, the target to which the probe of the second fluorescence wavelength is assigned, or a region containing the target, is used as a control. In one aspect, the control is a region with a relatively small CNV.

That is, as illustrated in FIG. 4, the PCR reaction system includes a plurality of probes including fluorescent materials with different fluorescence wavelengths, respectively, and the probes are assigned to different regions on the DNA sample, respectively. The region in this example is the entire length of each chromosome. The region may be part of each chromosome or may be longer than one chromosomes.

In one aspect illustrated in FIG. 4, each region contains one target. In an aspect different from FIG. 4, the region may contain two or more targets. In any case, fluorescence of fluorescence wavelengths assigned to the corresponding regions is detected from one reaction compartment (reaction droplet).

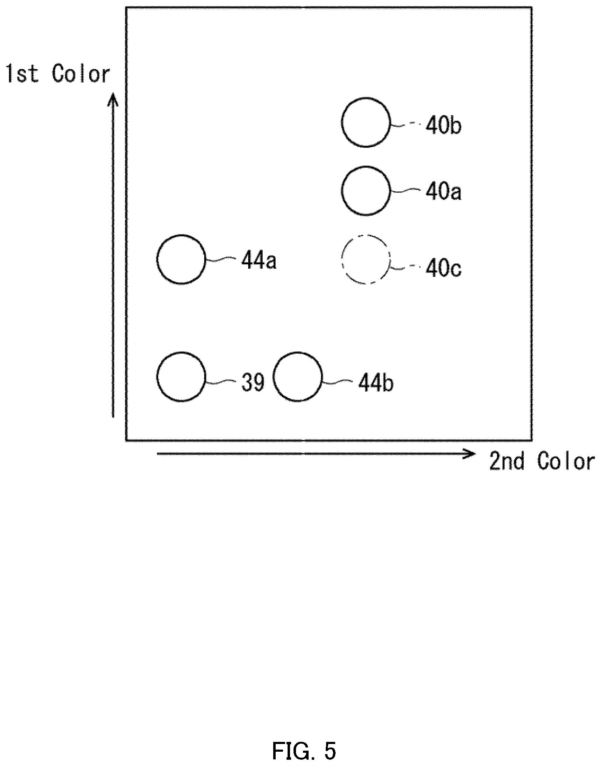

Under ideal reaction conditions for the aspect illustrated in FIG. 4, when chromosome 21 is triploid in its entirety or in a portion thereof to be detected, the intensity of the first fluorescence wavelength is 1.5 times the intensity of the first fluorescence wavelength of the case where chromosome 21 is diploid in its entirety or in a portion thereof to be detected. When chromosome 21 is monoploid in its entirety or in a portion thereof to be detected, the intensity of the first fluorescence wavelength is 0.5 times the intensity of the first fluorescence wavelength of the case where chromosome 21 is diploid in its entirety or in a portion thereof to be detected. This situation is illustrated in FIG. 5.

FIG. 5 illustrates a scatter plot of detection events of fluorescence emitted from reaction compartments (reaction droplets). The vertical axis represents the first fluorescence wavelength assigned to chromosome 21, and the horizontal axis represents the second fluorescence wavelength assigned to chromosome 18. Each circle indicates the vicinity of the center of the fluorescence intensity distribution of the detection events on the corresponding plot. Since most of the events are accumulated within this circle, the accumulated events are hereinafter referred to as a "cluster." Cluster 39 indicates the events whose fluorescence intensity does not differ from the background at either fluorescence wavelength. Clusters 40a to 40c indicate the events whose fluorescence intensities differ from the background at both fluorescence wavelengths.

Regarding cluster 40a illustrated in FIG. 5, the intensity of the first fluorescence wavelength indicates that chromosome 21 is diploid in its entirety or in a portion thereof to be detected. In addition, the intensity of the second fluorescence wavelength indicates that chromosome 18 is diploid in its entirety or in a portion thereof to be detected.

Regarding cluster 40b illustrated in FIG. 5, the intensity of the first fluorescence wavelength indicates that chromosome 21 is triploid in its entirety or in a portion thereof to be detected. In addition, the intensity of the second fluorescence wavelength indicates that chromosome 18 is diploid in its entirety or in a portion thereof to be detected.

Regarding cluster 40c illustrated in FIG. 5, the intensity of the first fluorescence wavelength indicates that chromosome 21 is monoploid in its entirety or in a portion thereof to be detected. In addition, the intensity of the second fluorescence wavelength indicates that chromosome 18 is diploid in its entirety or in a portion thereof to be detected.

In one aspect illustrated in FIG. 5, it is detected that points are shifted from the position of cluster 40a to the position of cluster 40b. In such a shift, the intensity of the first fluorescence wavelength increases. In such a shift, the intensity of the second fluorescence wavelength remains unchanged. Such a shift indicates the presence of single cells with chromosome 21 being triploid in its entirety or in a portion thereof to be detected, in the population of cells.

In another aspect illustrated in FIG. 5, it is detected that points are shifted from the position of cluster 40a to the position of cluster 40c. In such a shift, the intensity of the first fluorescence wavelength decreases. In such a shift, the intensity of the second fluorescence wavelength remains unchanged. Such a shift indicates the presence of single cells with chromosome 21 being monoploid in its entirety or in a portion thereof to be detected, in the population of cells.

The magnitude of the shifts on the scatter plot may be estimated by preliminary experiments. The ploidy of cells in each cluster on the scatter plot may be estimated by preliminary experiments.

The description returns to FIG. 3 to explain clusters 44a and 44b illustrated in FIG. 5. When generating droplets 33a, cell-free nucleic acid 32 may be involved in one of the droplet. Cell-free nucleic acid 32 is, for example, a fragment of genomic DNA released from a broken cell. An amplicon may also be generated from droplet 33b containing such cell-free genomic DNA by PCR.

In the scatter plot illustrated in FIG. 5, amplicons derived from such cell-free molecules generate clusters 44a and 44b. The signals of amplicons derived from cell-free molecules are less likely to coincide with cluster 40a. This is because it is unlikely that two or more targets on different regions are amplified simultaneously within a droplet derived from a cell-free molecule. Moreover, the copy number of a target of a droplet containing a cell-free molecule is usually one.

The description returns to FIG. 4. As illustrated in FIG. 4, fluorescent materials with different fluorescence wavelengths are used in the different regions, respectively. In other words, a probe of the second fluorescence wavelength is used as a control. As a result, a reaction droplet in which genomic DNA is amplified can be distinguished from a droplet in which only contaminating cell-free nucleic acid is amplified, as illustrated in FIG. 5.

That is, in the example illustrated in FIG. 4, the PCR reaction system includes a plurality of probes, respectively including fluorescent materials with fluorescence wavelengths different from each other, respectively assigned to different regions on the DNA sample. In addition, the region includes one or more of the above-described targets. In the step of quantifying the amplicons, the reaction compartment in which the target contained in the DNA sample is amplified is distinguished from a reaction compartment in which only a contaminating cell-free nucleic acid is amplified by detecting fluorescence of the plurality of wavelengths from one reaction compartment.

The type of fluorescent material (fluorophore dye) and quencher added to the probe is not limited. For example, the fluorescent material with the first fluorescence wavelength is FAM (trademark, Fluorescein amidite) and the fluorescent material with the second fluorescence wavelength is HEX (trademark, Hexachloro-fluorescein).

### Increased targets and enhanced signal

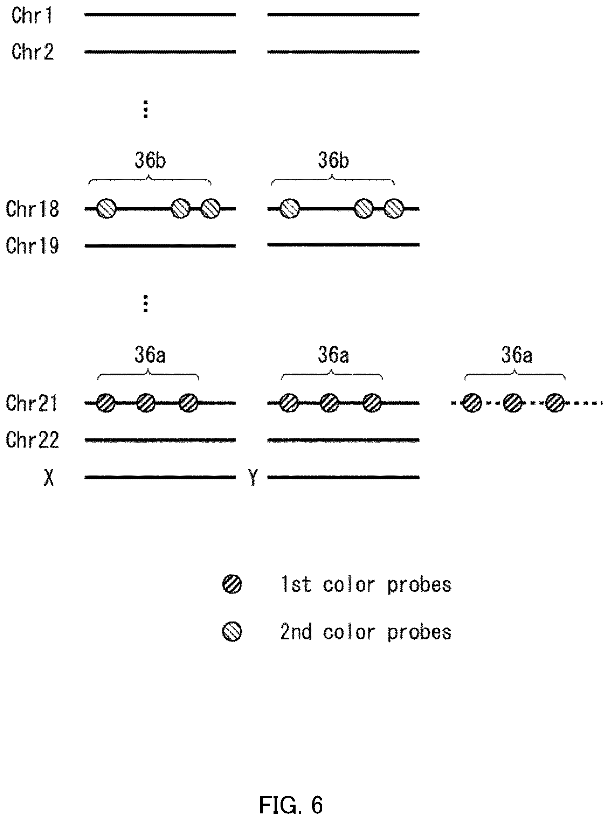

FIG. 6 illustrates an example in which a plurality of types of probes are assigned to each color. Differences from FIG. 4 will be described below. Three types of probes 36a (1st color) each including a fluorescent material with the first fluorescence wavelength are assigned to three types of targets on chromosome 21, respectively. Three types of probes 36b (2nd color) each including a fluorescent material with the second fluorescence wavelength are assigned to three types of targets on chromosome 18, respectively. These chromosome types are exemplary only. The PCR reaction system includes a plurality of probes, including fluorescent materials with different fluorescence wavelengths, assigned to different regions on the DNA sample, respectively. The targets of these probes have sequences different from each other. These probes are mixed together in the droplet.

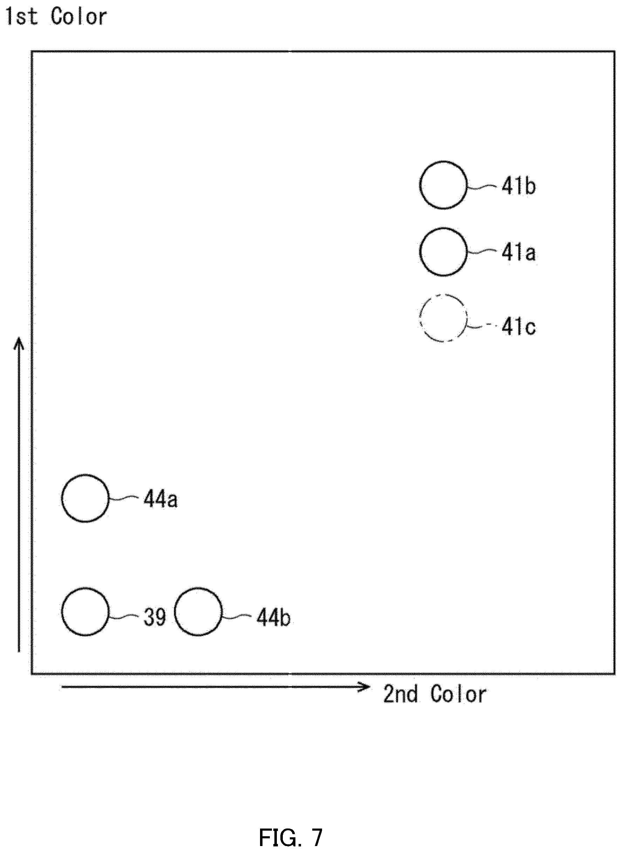

FIG. 7 illustrates a scatter plot of detection events of fluorescence emitted from reaction compartments (reaction droplets). Clusters 41a to 41c indicate the events whose fluorescence intensities differ from the background at both fluorescence wavelengths. FIG. 7 illustrates shifts similar to those of FIG. 5.

Regarding cluster 41a illustrated in FIG. 7, the intensity of the first fluorescence wavelength indicates that chromosome 21 is diploid in its entirety or in a portion thereof to be detected. In addition, the intensity of the second fluorescence wavelength indicates that chromosome 18 is diploid in its entirety or in a portion thereof to be detected.

Regarding cluster 41b illustrated in FIG. 7, the intensity of the first fluorescence wavelength indicates that chromosome 21 is triploid in its entirety or in a portion thereof to be detected. In addition, the intensity of the second fluorescence wavelength indicates that chromosome 18 is diploid in its entirety or in a portion thereof to be detected.

Regarding cluster 41c illustrated in FIG. 7, the intensity of the first fluorescence wavelength indicates that chromosome 21 is monoploid in its entirety or in a portion thereof to be detected. In addition, the intensity of the second fluorescence wavelength indicates that chromosome 18 is diploid in its entirety or in a portion thereof to be detected.

In one aspect illustrated in FIG. 7, it is detected that points are shifted from the position of cluster 41a to the position of cluster 41b. In such a shift, the intensity of the first fluorescence wavelength increases. In such a shift, the intensity of the second fluorescence wavelength remains unchanged. Such a shift indicates the presence of single cells with chromosome 21 being triploid in its entirety or in a portion thereof to be detected, in the population of cells.

In another aspect illustrated in FIG. 7, it is detected that points are shifted from the position of cluster 41a to the position of cluster 41c. In such a shift, the intensity of the first fluorescence wavelength decreases. In such a shift, the intensity of the second fluorescence wavelength remains unchanged. Such a shift indicates the presence of single cells with chromosome 21 being monoploid in its entirety or in a portion thereof to be detected, in the population of cells.

The magnitude of the shifts on the scatter plot may be estimated by preliminary experiments.

Clusters 44a and 44b illustrated in FIG. 7 are similar to those illustrated in FIG. 5. These clusters can be distinguished from clusters 41a to 41c by using a technique of detecting probes with different fluorescence wavelengths within the same droplet. This is also similar to FIG. 5.

The description returns to FIG. 6. Three probes each including a fluorescent material with the first fluorescence wavelength are assigned to each chromosome 21. Similarly, three probes each including a fluorescent material with the second fluorescence wavelength are assigned to each chromosome 18. Therefore, under ideal reaction conditions, the fluorescence intensity of each color in the reaction droplet increases in proportion to the number of targets in the region. In addition, increasing the number of targets in a single reaction droplet averages out the variation in PCR efficiency and reduces the variation in fluorescence intensity between reaction droplets.

With reference to FIG. 7, clusters 41a to 41c are more likely to be separated from cluster 39, cluster 44a, and cluster 44b.

In the present embodiment, each region contains a plurality of targets, and a plurality of probes including fluorescent materials with identical fluorescence wavelengths are assigned to the plurality of targets, respectively. In addition, for quantifying amplicons for each region, the amplicons are quantified by measuring fluorescence from the plurality of probes collectively by color, regardless of the difference between the targets. This configuration enhances the fluorescence from the target assigned regions. Therefore, it is easier to separate the signal of a target from the background noise.

In one aspect, the number of targets in one region is 1 to 1,000. In one aspect, the number of targets in one region is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100. The number of targets may be the same or different between regions. In each region, as many types of probes as there are targets are used. The same fluorescence wavelength is assigned to these probes.

In one aspect, a single combination of primer pair and probe specifies only one target on the genomic DNA. Using n types of probes in each region can detect n targets in this region.

### Assignment of three or more colors to three or more regions

In the above embodiment, two fluorescence wavelengths are used. Further, the number of regions may be increased, and another fluorescence wavelengths may be assigned to an additional regions. In one aspect, a third fluorescence wavelength may be assigned to a chromosome other than chromosomes 18 and 21.

In one aspect, the number of sets of region and fluorescence wavelength is 2, 3, 4, 5, 6, 7, 8, 9, 10, 11, 12, 13, 14, 15, 16, 17, 18, 19, 20, 21, 22, 23, or 24. In another aspect, the number of sets of region and fluorescence wavelength is one.

The type of fluorescent material (fluorophore dye) and quencher added to the probe is not limited. Examples of the fluorescent material include FAM, HEX, VIC, TAMRA, ROX, Cy5 and Cy5.5 (all trademarks).

### Test example 1: Quantitation at plateau

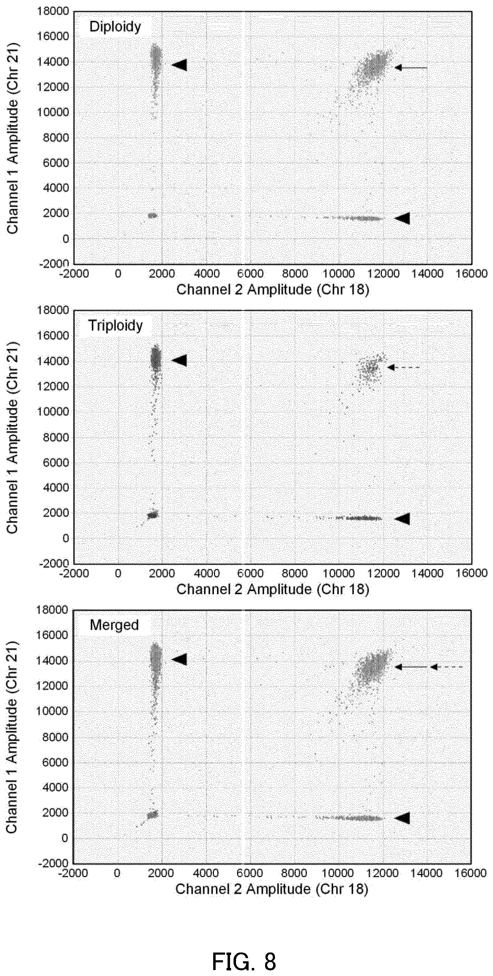

FIG. 8 illustrates scatters of droplets in a plateau. One type of probe including a fluorescent material (FAM) with a first fluorescence wavelength was assigned to one target on chromosome 21. In addition, one type of probe including a fluorescent material (HEX) with a second fluorescence wavelength was assigned to one target on chromosome 18. The probes were mixed together in one droplet.

The number of PCR cycles was 40. The PCR reaction reached a plateau through an exponential amplification phase. The reaction was saturated. FIG. 8 illustrates the scatters after single cell-droplet PCR.

The upper part of FIG. 8 illustrates the results of single cell-droplet PCR performed on a human cultured cell line GM22948. In the human cultured cell line GM22948, chromosomes 21 and 18 are euploid, that is, diploid (solid arrow, Diploidy). A solid arrow indicates the center of a cluster. An arrowhead indicates a cluster of cell-free DNA. The same applies to the following drawings.

The middle part of FIG. 8 illustrates the results of single cell-droplet PCR performed on a human cultured cell line AG17487. In the human cultured cell line AG17487, chromosome 21 is triploid (dashed arrow, Triploidy) and chromosome 18 is euploid.

The lower part of FIG. 8 illustrates the result of adding the scatters of both lines together. No difference was found in the signal intensity of droplets between diploidy and triploidy of chromosome 21.

### Test example 2: Quantification during exponential amplification phase

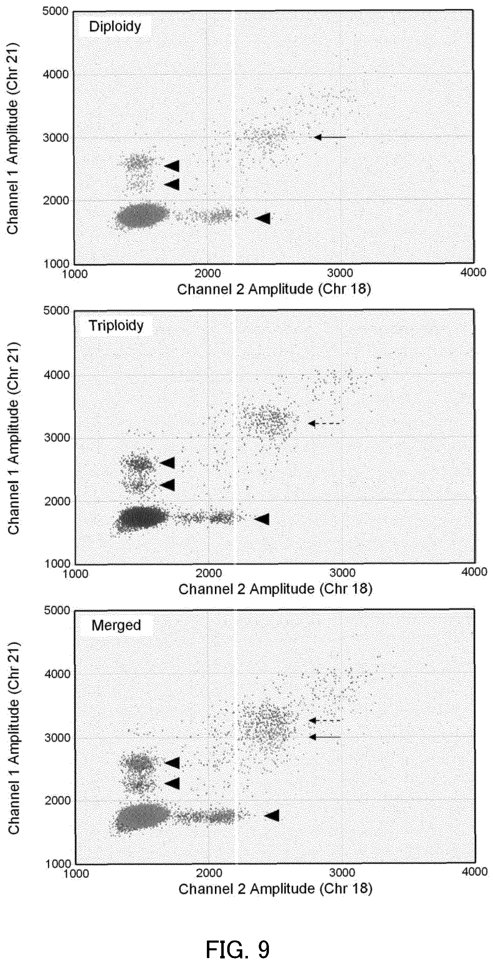

FIG. 9 illustrates scatters of droplets during an exponential amplification phase. The number of PCR cycles was 24, which is different from that of Test example 1. The PCR reaction was in the exponential amplification phase. Other conditions and the like were the same as Test Example 1.

The upper part of FIG. 9 illustrates the results of single cell-droplet PCR performed on the human cultured cell line GM22948.

The middle part of FIG. 9 illustrates the results of single cell-droplet PCR performed on the human cultured cell line AG17487.

The lower part of FIG. 9 illustrates the result of adding the scatters of both lines together. A difference was found in the signal intensity of droplets between diploidy and triploidy of chromosome 21. Quantifying droplet fluorescence during the exponential amplification phase can distinguish between diploidy and triploidy. Therefore, in the test examples below, the fluorescence intensity of droplets was measured during the exponential amplification phase.

In each scatter of FIG. 9, a plurality of events that are located above and to the right of a cluster of diploid chromosome 18 and diploid or triploid chromosome 21 is considered to indicate the signals of droplets each containing a plurality of cells.

### Test example 3: Enhancing fluorescence

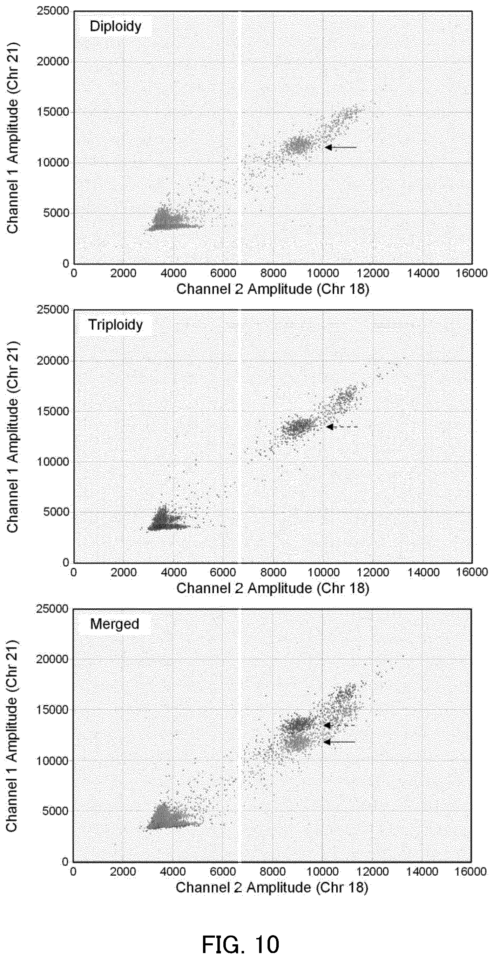

FIG. 10 illustrates scatters of droplets with enhanced fluorescence. Eight types of probes each including a fluorescent material with a first fluorescence wavelength were assigned to chromosome 21, which is different from that of Test example 2. One of the above types of probes including the fluorescent material with the first fluorescence wavelength was assigned to each of eight targets on chromosome 21. Eight types of probes each including a fluorescent material with a second fluorescence wavelength were assigned to chromosome 18. One of the above types of probes including the fluorescent material with the second fluorescence wavelength was assigned to each of eight targets on chromosome 18. Other conditions and the like were the same as Test Example 2.

The upper part of FIG. 10 illustrates the results of single cell-droplet PCR performed on the human cultured cell line GM22948.

The middle part of FIG. 10 illustrates the results of single cell-droplet PCR performed on the human cultured cell line AG17487.

The lower part of FIG. 10 illustrates the result of adding the scatters of both lines together. The cluster of diploid chromosome 21 and the cluster of triploid chromosome 21 were more clearly separated. The detection of the shift itself of the cluster became easy. This is because the fluorescence of a droplet was enhanced and the variation in fluorescence intensity was reduced by increasing the number of targets within one region corresponding to probes of the same fluorescence wavelength. In the test examples below, fluorescence was enhanced by assigning a plurality of targets to one region.

In each scatter of FIG. 10, a plurality of events that are located above and to the right of a cluster of diploid chromosome 18 and diploid or triploid chromosome 21 is considered to indicate the signals of droplets each containing a plurality of cells.

### Test example 4: Detection of CNV in heterogeneous cell population

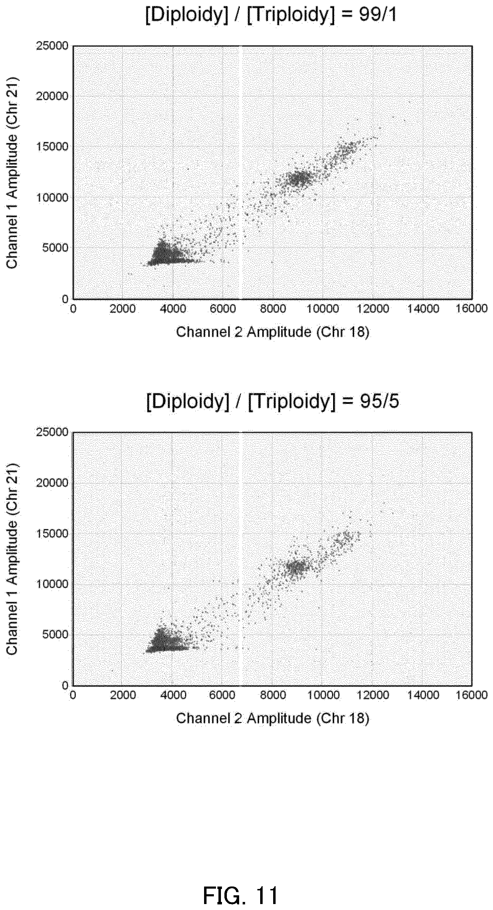

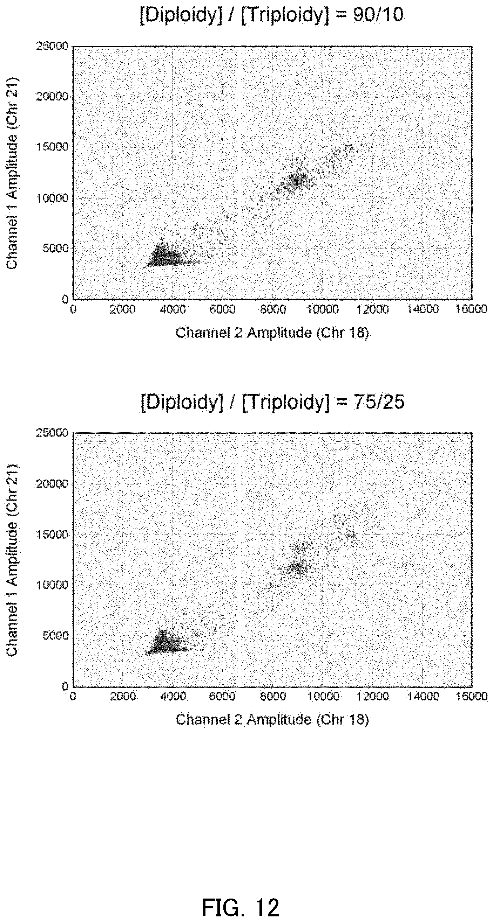

FIGS. 11 and 12 illustrates scatters of droplets in heterogeneous cell populations. The human cultured cell line GM22948 (Diploidy) and the human cultured cell line AG17487 (Triploidy) were mixed to obtain heterogeneous cell populations. The mixing ratio was set from 99/1 to 75/25. Single cell-droplet PCR was performed on these cell populations. Other conditions and the like were the same as Test Example 3. The cluster shift from diploidy to triploidy reflected the cell number ratio.

### Test example 5: Cluster shift from diploid to monoploid

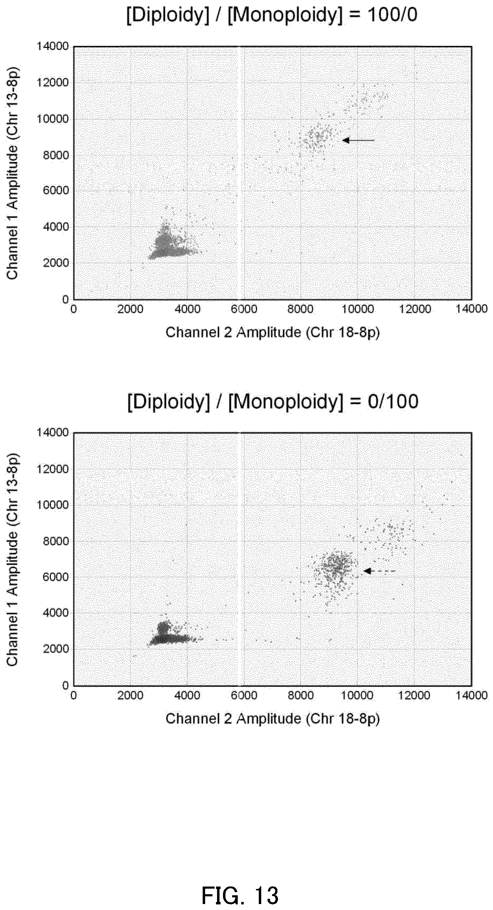

FIG. 13 illustrates scatters of droplets in homozygous cell populations. Chr13-8p on the vertical axis indicates that 8 types of probes are assigned to 8 targets on chromosome 13, respectively. Chr18-8p on the horizontal axis indicates that 8 types of probes are assigned to 8 targets on chromosome 18, respectively. The probes were mixed together in one droplet.

The upper part of FIG. 13 illustrates the results of single cell-droplet PCR performed on the human cultured cell line GM22948. In the human cultured cell line GM22948, chromosomes 13 and 18 are euploid, that is, diploid (solid arrow, Diploidy).

The lower part of FIG. 13 illustrates the results of single cell-droplet PCR performed on a human cultured cell line NCI-H929. In the human cultured cell line NCI-H929, chromosome 13 is monoploid (dashed arrow, Monoploidy) and chromosome 18 is euploid.

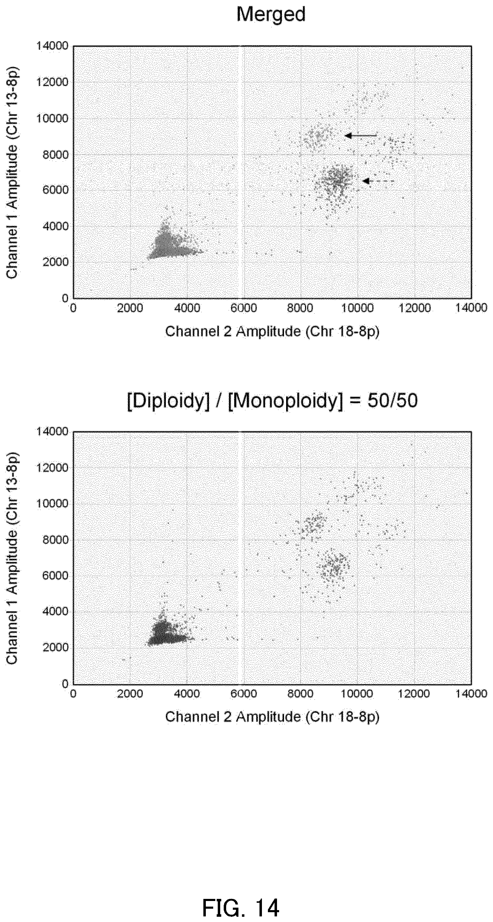

The upper part of FIG. 14 illustrates the result of adding the scatters of both lines together. A difference was found in the signal intensity of droplets between diploidy and monoploidy of chromosome 13. Quantifying droplet fluorescence during the exponential amplification phase can distinguish between diploidy and monoploidy.

The lower part of FIG. 14 illustrates a scatter of droplets in a heterogeneous cell population. The human cultured cell line GM22948 (Diploidy) and the human cultured cell line NCI-H929 (Monoploidy) were mixed to obtain a heterogeneous cell population. The mixing ratio was set to 50/50. Single cell-droplet PCR was performed on the cell population. The cluster shift from diploidy to monoploidy reflected the cell number ratio.

### Test example 6: Enhancing fluorescence

A PCR reaction solution was prepared by adding 2 µL of a cell suspension containing 10,000 cells (line GM22948 or line GM13721) and PBS to the PCR premix containing sodium dodecyl sulfate. The concentrations of primers, probes, and the like in the PCR reaction solution are as follows.
- 1X ddPCR Multiplex Super Mix (BioRad Laboratories, Inc.)
- 500 nM/575 nM Chromosome 13-FAM probe
- 1 µM Chromosome 13 primer
- 1000 nM/1250 nM Chromosome 18-HEX probe
- 1 µM Chromosome 18 primer
- Distilled water (for filling up)

The above PCR reaction solution was made into droplets by using QX200 Droplet Generator (BioRad Laboratories, Inc.), and cells were lysed in the respective droplets. Subsequently, PCR was performed under general conditions within each droplet. The number of PCR cycles was 23. The PCR reaction was in the exponential amplification phase.

In the present test example, 1, 5, 10, or 23 types of probes each including a fluorescent material (FAM) with the first fluorescence wavelength were assigned to chromosome 13. One of the above types of probes including the fluorescent material with the first fluorescence wavelength was assigned to each of 1, 5, 10, or 23 targets on chromosome 13. When the plurality of types of probes are used, the concentrations of the probes are the same, and the concentrations of the above probes (1, 5 or 10 types: 500 nM and 23 types: 575 nM) are the total concentrations of the plurality of types of probes. In addition, 1, 5, 10, or 25 types of probes each including a fluorescent material (HEX) with the second fluorescence wavelength were assigned to chromosome 18. One of the above types of probes including the fluorescent material with the second fluorescence wavelength was assigned to each of 1, 5, 10, or 25 targets on chromosome 18. When the plurality of types of probes are used, the concentrations of the probes are the same, and the concentrations of the above probes (1, 5 or 10 types: 1,000 nM and 25 types: 1,250 nM) are the total concentrations of the plurality of types of probes. The probes were mixed together in one droplet.

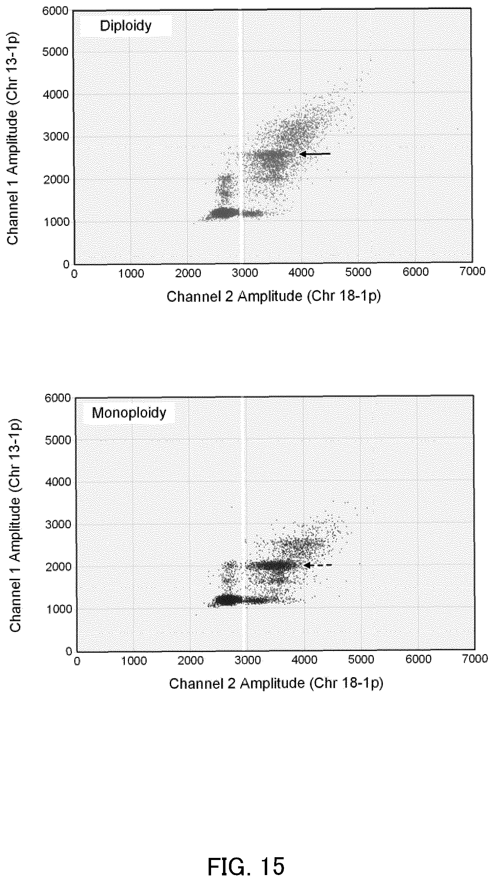

FIGS. 15 to 18 illustrates scatters of droplets during an exponential amplification phase.

The upper parts of FIGS. 15 to 18 illustrate the results of single cell-droplet PCR performed on the human cultured cell line GM22948. In the human cultured cell line GM22948, chromosomes 13 and 18 are euploid, that is, diploid (solid arrow, Diploidy). A solid arrow indicates the center of a cluster.

The lower parts of FIGS. 15 to 18 illustrate the results of single cell-droplet PCR performed on the human cultured cell line GM13721. In the human cultured cell line GM13721, a partial region of one long arm of chromosome 13 is monoploid (broken arrow, Monoploidy), and chromosome 18 is euploid. All probes including the fluorescent material (FAM) with the first fluorescence wavelength are assigned to the defective region of chromosome 13.

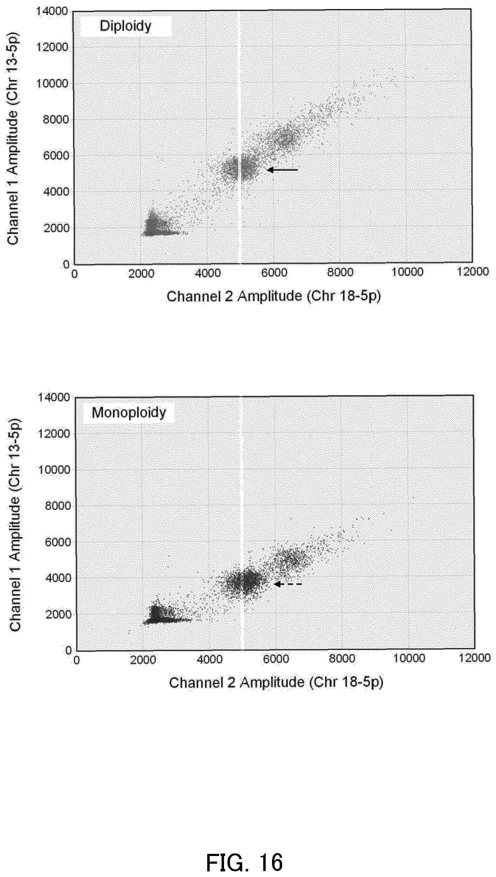

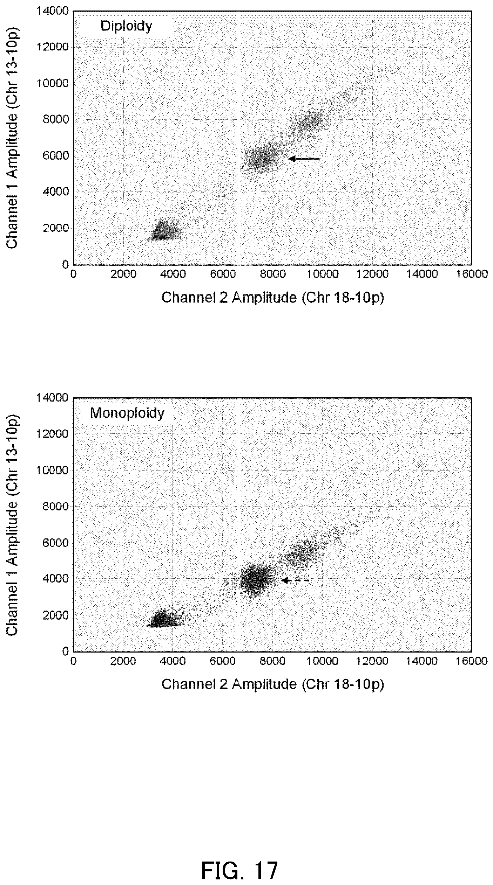

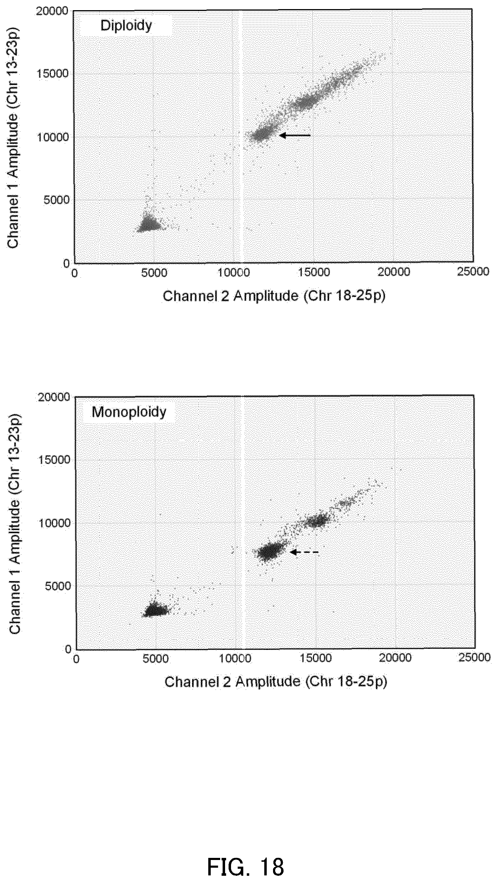

FIG. 15 illustrate the results when one type of probe was assigned to each of chromosome 13 and chromosome 18. FIG. 16 illustrates the results when five types of probes were assigned to each of chromosome 13 and chromosome 18. FIG. 17 illustrates the results when 10 types of probes were assigned to each of chromosome 13 and chromosome 18. FIG. 18 illustrates the results when 23 types of probes were assigned to chromosome 13 and 25 types of probes were assigned to chromosome 18.

In each scatter of FIGS. 15 to 18, a plurality of events that are located above and to the right of a cluster of diploid chromosome 18 and monoploid or diploid chromosome 13 is considered to indicate the signals of droplets each containing a plurality of cells.

As clearly seen from the difference in the positions of the solid and dashed arrows in these scatters, increasing the number of targets within one region (the targets correspond to probes of the same fluorescence wavelength) can determine copy number differences more easily.

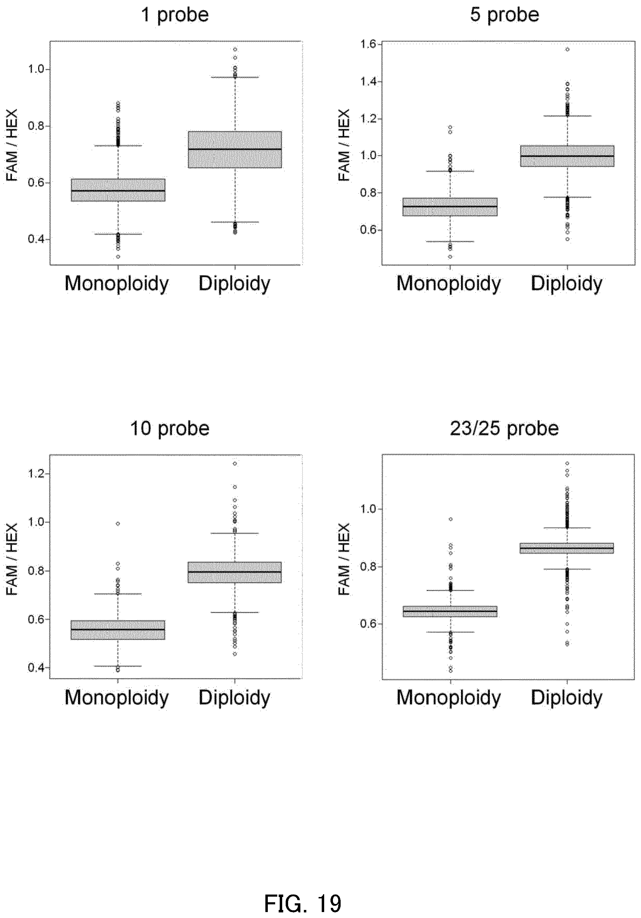

Subsequently, for each scatter, the ratio (FAM/HEX) of fluorescence intensity at the first fluorescence wavelength with respect to fluorescence intensity at the second fluorescence wavelength was calculated for droplets each containing one cell, and summarized in a boxplot. FIG. 19 illustrates boxplots for respective scatters.

In all the graphs of FIG. 19, for cells with monoploid chromosome 13 and cells with diploid chromosome 13, boxes showing third quartile and first quartile do not overlap each other; thus monoploid and diploid cells can be distinguished from each other. In particular, when 23 types of probes were used for chromosome 13 and 25 types of probes were used for chromosome 18, even the whiskers did not overlap each other.

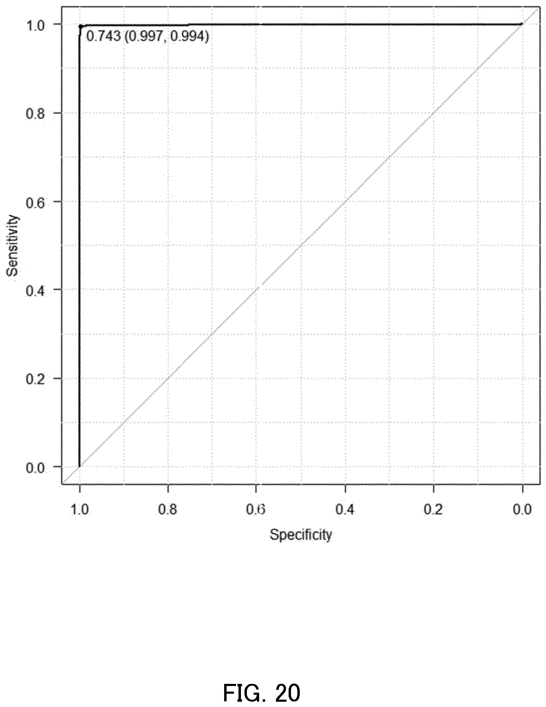

FIG. 20 illustrates the results of ROC analysis of the ability to detect the line GM13728 (cells with monoploid chromosome 13) from a cell population containing the line GM22948 (cells with diploid chromosome 13) and the line GM13728 (cells with monoploid chromosome 13) by using "FAM/HEX" when using 23 types of probes as an explanatory variable. The Youden Index was 0.743. The sensitivity was 0.997 (99.7%) and the specificity was 0.994 (99.4%) with cut off by the Youden Index. In addition, AUC was 0.997 (95% CI: 0.996 to 0.999), which was a very high value.

### Internal standard

In one aspect, ploidy measurements are made with an internal standard. In one aspect, cells that are euploid in the region to be detected are the internal standard for the CNV. For example, in Test Example 4 and the second half of Test Example 5, the copy number of the region to be detected in a heterogeneous cell population was measured. With respect to euploid clusters, the presence of clusters with CNVs was used to determine whether the clusters are triploidy or monoploidy.

In a heterogeneous cell population, the proportion of cells that are euploid in the target assigned region is greater than 0% and less than 100%. The ratio of euploid cells in the heterogeneous cell population is, for example, 0.01, 0.02, 0.03, 0.04, 0.05, 0.1, 0.2, 0.3, 0.4, 0.5, 1, 2, 3, 4, 5, 10, 15, 20, 25, 30, 40, 50, 60, 70, 75, 80, 85, 90, 95, 96, 97, 98, 99, 99.5, 99.6, 99.7, 99.8, 99.9, 99.95, 99.96, 99.97, 99.98, or 99.99%.

In a heterogeneous cell population, the proportion of cells with CNVs of interest in the target assigned region is greater than 0% and less than 100%. The ratio of cells with CNVs in the heterogeneous cell population is, for example, 99.99, 99.98, 99.97, 99.96, 99.95, 99.9, 99.8, 99.7, 99.6, 99.5, 99, 98, 97, 96, 95, 90, 85, 80, 75, 70, 60, 50, 40, 30, 25, 20, 15, 10, 5, 4, 3, 2, 1, 0.5, 0.4, 0.3, 0.2, 0.1, 0.05, 0.04, 0.03, 0.02, or 0.01%.

### External standard

In Test Examples 1 to 3 and the first half of Test Example 5, the copy number of the region to be detected in a homozygous cell population for polyploidy was measured. The CNVs of these cells were previously known. The following means can provide a copy number standard for the region to be detected in cells whose CNV is not identified in advance.

That is, in one aspect, ploidy measurements are made with an external standard. For example, in another emulsion system that is not mixed with the sample, PCR is performed simultaneously or before or after the sample measurement by using genomic DNA of cells whose ploidy is known in advance as a template, and the fluorescence intensity of the amplicon is measured. The cluster coordinates obtained from the fluorescence intensity are compared with the sample cluster coordinates.

Examples of such test examples with external standards are illustrated in the upper part (Diploidy) and the middle part (Triploidy) of FIG. 9, the upper part (Diploidy) and the middle part (Triploidy) of FIG. 10, and the upper part (Diploidy) and the lower part (Monoploidy) of FIG. 13.

The following is an example of measuring the polyploidy of any one of the chromosomes of a cancer patient's tumor cells. As the treatment progresses smoothly, the originally present chromosomal mutations may disappear in all the cells to be measured. In this case, the region on the chromosome to be measured becomes euploid. Therefore, according to the above measurement method, an aneuploid cluster on the scatter disappears. In other words, only an euploid cluster remains among the various clusters.

On the other hand, when the disease of cancer patients significantly progresses, it is assumed that all the cells to be measured have chromosomal mutations. Alternatively, it is assumed that there will be significantly more aneuploid cells than euploid cells, and euploid cells may become undetectable. According to the above measurement method, the euploid cluster on the scatter disappear. In other words, only an aneuploid cluster will be detected among the various clusters.

Such an aneuploid cluster becomes a homogeneous population of cells with a specific ploidy in the region to be measured. Alternatively, the cluster becomes a heterogeneous population of cells with various ploidy.

In one aspect, genome polyploidy of tumor cells from cancer patients is measured with an external standard of ploidy. Using an external standard makes possible to measure cell ploidy without relying on euploid cells (healthy, non-cancerous cells) that should have been contained in a cell population as an internal standard. More accurate information on these ploidy is useful in determining the need for cancer treatment or in the diagnosis of complete remission.

The following is an example as a part of prenatal diagnosis in which fetal cells in amniotic fluid are to be measured. When fetal cells in the amniotic fluid are free of maternal cell contamination, the normal population of such cells would contain only fetal cells. Such a cell population is homogenous with respect to genome polyploidy.

In one aspect, genome ploidy of fetal cells is measured with an external ploidy standard. Using an external standard makes possible to measure cell ploidy without relying on euploid cells (in this case, cells of maternal origin) that should have been contained in a cell population as an internal standard.

When it can be confirmed in advance that fetal cells have been obtained from the amniotic fluid with 100% purity, using the above measurement method makes possible to examine whether the fetal cell population is heterogeneous or homogeneous with respect to genome ploidy. That is, the presence or absence of ploidy mosaicism in chromosomes of fetal cells can be checked.

In the present embodiment, there is a very clear linear relationship between the copy number of the genomic region to be measured and the center of fluorescence intensity of the cluster. Therefore, the obtainment of the fluorescence intensity from the external standard cells is not necessary each time the sample is measured. In other words, the ploidy of the region of the genome of the sample subjected to the measurement can be determined by comparing the fluorescence intensity information of the external standard obtained in advance with the fluorescence intensity information of the sample.

### Test example 7: Setting of cutoff value as external standard

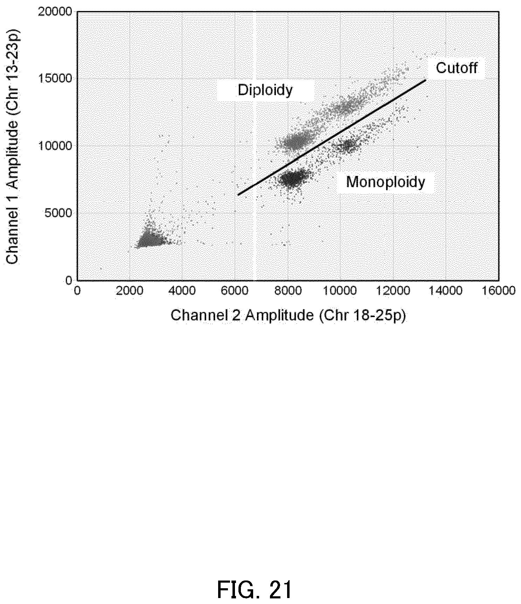

FIG. 21 illustrates a scatter including a straight line corresponding to the cutoff value to be set in the present test example. In the present test example, as illustrated in FIG. 21, set as an external standard is a cutoff value that separates cells with a partial region of one long arm of chromosome 13 being monoploid from cells with diploid chromosome 13.

A PCR reaction solution was prepared by adding 10,000 cells (line GM22948) and 2 µL of a cell suspension containing PBS to the PCR premix containing sodium dodecyl sulfate. The concentrations of primers, probes, and the like in the PCR reaction solution are as follows.
- 1X ddPCR Multiplex Super Mix (BioRad Laboratories, Inc.)
- 25 nM chromosome 13-FAM probe × 23 types (total concentration: 575 nM)
- 1 µM Chromosome 13 primer × 23 types
- 50 nM Chromosome 18-HEX probe × 25 types (total concentration: 1,250 nM)
- 1 µM Chromosome 18 primer × 25 types
- Distilled water (for filling up)

The above PCR reaction solution was made into droplets by using QX200 Droplet Generator (BioRad Laboratories, Inc.), and cells were lysed in the respective droplets. Subsequently, PCR was performed under general conditions within each droplet. The number of PCR cycles was 23. The PCR reaction was in the exponential amplification phase. Using the 23 separately prepared PCR reaction solutions, PCR was performed separately.

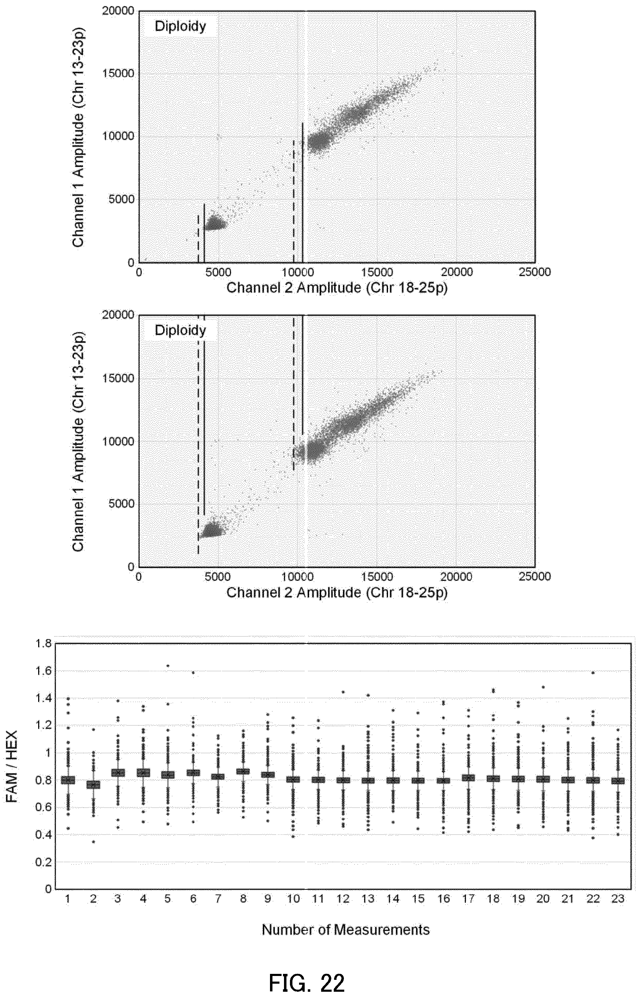

FIG. 22 illustrates cluster displacement for each measurement. The upper and middle parts of FIG. 22 illustrate the results of single cell-droplet PCR performed on the human cultured cell line GM22948. In the human cultured cell line GM22948, chromosomes 13 and 18 are euploid, that is, diploid (solid arrow, Diploidy). As illustrated in the drawings, when the same probe is used on the same cell line for a plurality of measurements, cluster positions may differ between different measurements. The direction in which all cluster moves is the same in each measurements.

The lower part of FIG. 22 is a graph showing a variation in measured values when the measurement is performed 23 times. This graph uses FAM/HEX as the measured value. As can be seen from this graph, measurement results vary even when the same probe is used for the same cell line in the measurement. In order to set an appropriate cutoff value, it is preferable to set the cutoff value after correcting this variation.

Therefore, in the present test example, the following medians of the negative cluster (the cluster located at the lower left in the scatter) were calculated for each of the 23 measurement results: the median of fluorescence intensity of the first fluorescence wavelength (FAM) (hereinafter also referred to as "FAM measurement value") and the median of fluorescence intensity of the second fluorescence wavelength (HEX) (hereinafter also referred to as "HEX measurement value"). The average value of the 23 FAM measurement values was used as the FAM reference value for the negative cluster, and the average value of the 23 HEX measurement values was used as the HEX reference value for the negative cluster. Subsequently for each of the 23 measurement results, the calculation results "(FAM reference value + α) / (FAM measurement value + α)" and "(HEX reference value + α) / (HEX measurement value + α)" were used as correction values for correcting cluster shifts (variation in measurement results). Herein, α is a numeric value arbitrarily set in order to bring the ratio of "FAM reference value / FAM measurement value" and "HEX reference value / HEX measurement value" close to the true fluorescence intensity ratio in consideration of the fluorescence intensity detection limit of the measuring device. Herein, α is set to 2,000. All the measured values were multiplied by the obtained FAM correction value and HEX correction value to correct variations in the measurement results.

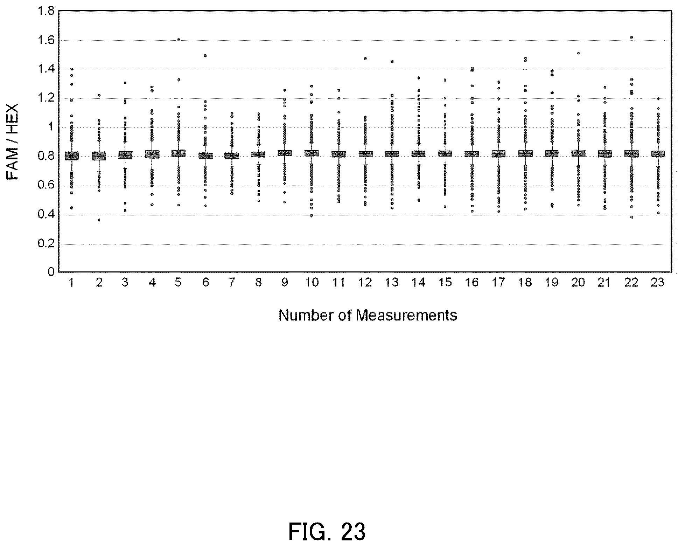

FIG. 23 illustrates a graph showing variations in the corrected measured values. The measurement number corresponds to that of the graph in the lower part in FIG. 22. It is apparent that the variation in measurement results (cluster shift) is significantly reduced. A cutoff value was set by using these corrected measured values.

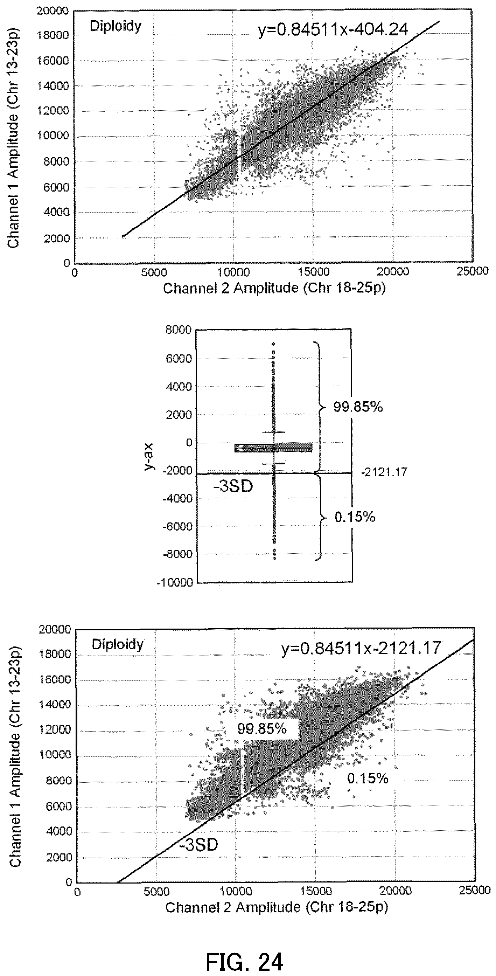

FIG. 24 is a diagram for explaining calculation of a cutoff value using corrected measured values. The upper part of FIG. 24 is a scatter showing 95,341 corrected measured values. A linear function equation (y=ax+b) that approximates the distribution of all the measured values was obtained by the standard major axis regression, obtaining the equation y = 0.84511x - 404.24. For integrating the fluorescence intensity of the first fluorescence wavelength (FAM) and the fluorescence intensity of the second fluorescence wavelength (HEX) for each measurement, the value of b for each measured value was obtained by substituting the measured values into x (fluorescence intensity of the second fluorescence wavelength (HEX)) and y (fluorescence intensity of the first fluorescence wavelength (FAM)) of the equation b = y - 0.84511x obtained by conversion of the above linear equation. The graph in the middle part of FIG. 24 illustrates the distribution of the calculated 95,341 b values. In this embodiment, with an emphasis on specificity, the value corresponding to - 3SD (standard deviation) of the distribution shown in this graph was used as a cutoff value for separating cells with monoploid chromosome 13 from cells with diploid chromosome 13. The graph in the lower part of FIG. 24 is a scatter containing a straight line (y = 0.84511x - 2121.17) representing the cutoff value. Among the 95,341 measured values for cells with diploid chromosome 13, 99.85% of the measured values are located above this straight line.

Table 1 shows the results of determining the measurement results using the obtained cutoff value. The sensitivity was 84.1% and the specificity was 99.3% when detecting the line GM13721 (cells with a partial region of one long arm of chromosome 13 being monoploid) from the cell population containing the line GM22948 (cells with diploid chromosome 13) and the line GM13721. As described above, the cutoff value set in this embodiment emphasizes specificity, and it is of course possible to set the cutoff value with emphasis on sensitivity.

**Table 1**

| | | Cell line | |
|---|---|---|---|
| | | GM13721 monoploid | GM22948 diploid |
| Determination result | Monoploidy | 2740 | 22 |
| | Diploidy | 517 | 3235 |

The method of setting the cutoff value is not limited to the above examples. For example, ROC analysis may be used to calculate the cutoff value that maximizes sensitivity and specificity.

### Detection of mutation

In one aspect, detecting a CNV detects the presence of a single cell with a mutation in a population of cells. In one aspect, the mutation is a somatic or germline mutation. In one aspect, the mutation is a chromosomal or genetic mutation.

### Detection of chromosomal mutation

In one aspect, when DNA sample contains a set of genomic DNA in a single cell, the presence of a single cell with a chromosomal mutation is detected in the cell population by detecting a CNV from the results of quantification of PCR amplicons. In one aspect, the chromosomal mutation is at least one of the following: aneuploidy over the entire length of a chromosome, partial aneuploidy of a chromosome, gene amplification, and gene deletion. FIGS. 4 to 7 described above are directed to one aspect of the detection of aneuploidy over the entire length of a chromosome.

Gene amplification is the multiplication of a gene due to partial duplication of a chromosome. In one aspect, there is no limit to the number of times duplication occurs. Gene deletion is the loss of a gene due to partial deletion of a chromosome. In one aspect, the gene deletion is haplo- or null-type. In one aspect, gene amplification and deletion are polymorphism.

As described above, unless the cell population is dominated by cells with genomic aneuploidy, the cell population cell includes euploid cells. The cell population contains normal single cells with no chromosomal mutations at the location of a target genome. At least one of the single cells assigned to the respective reaction compartments (reaction droplets) described above have euploid chromosomes in the regions to be subjected to aneuploidy detection. On the scatter, a cluster of single cells with chromosomal mutations is shifted from the position of a cluster produced by normal single cells. Cluster 40a illustrated in FIG. 5 and cluster 41a illustrated in FIG. 7 described above are examples of such a shift. Cluster 40a functions as a type of internal standard.

In addition, even when the cell population is dominated by cells with genomic aneuploidy, the ploidy of the cluster can be absolutely quantified by using the external standard described above. The external standard can also be applied to a heterogeneous cell population consisting of euploid and aneuploid cells.

Detected is a reaction droplet that has an amplicon amount different from that of a reaction droplet including a single cell not having a chromosomal mutation at the location of a target genome. Hereinafter, this is referred to as an aneuploid droplet. Clusters 40b and 40c illustrated in FIG. 5 and clusters 41b and 41c illustrated in FIG. 7 described above are examples of such detection.

Examples of chromosomal mutations that result in aneuploid droplets are the following. That is, a single cell with a detectable chromosomal mutation contains, for example, genomic DNA with any one of the following chromosomal mutations.
- Aneuploidy of chromosome 21
- Aneuploidy of chromosome 18
- Aneuploidy of chromosome 13
- Aneuploidy of Y chromosome
- Aneuploidy of X chromosome
- Deletion of the 22q11.2 region on a long arm of chromosome 22
- Deletion of the 5q region on a short arm of chromosome 5
- Deletion of the 15q11-q13 region on a long arm of chromosome 15
- Amplification of a long arm of chromosome 1
- Deletion of a short arm of chromosome 17
- Deletion of a long arm of chromosome 13
- Deletion of a long arm of chromosome 4
- Deletion of a long arm of chromosome 5
- Deletion of a long arm of chromosome 7
- Amplification of chromosome 8
- Deletion of chromosome 11
- Aneuploidy of chromosome 12
- Deletion of a long arm of chromosome 20
- Deletion of a long arm of chromosome 19
- Deletion of chromosome 1
- Deletion of a long arm of chromosome 18
- Deletion of a short arm of chromosome 8
- Deletion of chromosome 4
- Amplification of a long arm of chromosome 8
- Deletion of a long arm of chromosome 16
- Amplification of a short arm of chromosome 5
- Amplification of a long arm of chromosome 3
- Deletion of a short arm of chromosome 3
- Deletion of a short arm of chromosome 9
- Gene amplification of MYCN gene
- Gene amplification of HER2 gene
- Gene amplification of MET gene

In one aspect, deletions exclude biallelic deletions, so-called nulls. In one aspect, a pair formed of a chromosome with a deletion part and a chromosome with a corresponding part being normal is to be detected.

In one aspect, data including information on whether or not an aneuploid droplet is detected is further generated. That is, data including information on whether or not a single cell with a chromosomal mutation is detected is generated. Selecting a cell population that generates single cells genetates useful data in specific diagnostic areas described below.

### Application to prenatal diagnosis

In one aspect, single cells are obtained from either amniotic fluid or maternal blood. Specifically, a population of bulk cells are obtained from amniotic fluid or maternal blood. The cell population includes fetal cells. In one aspect, fetal cells in the cell population are enriched. A cell population includes maternal cells. In one aspect, the maternal cell is euploid in the region on the genomic DNA to be detected. In one aspect, the fetal cell is a fetal nucleated red blood cell (fetal nucleated erythroblasts, fNRBC) circulating in maternal blood, and the maternal cell is a white blood cells circulating in maternal blood. In one aspect, the fetal cell is a fetal cell floating in amniotic fluid and the maternal cell is a maternal cell floating in amniotic fluid.

In one aspect, data is provided for the diagnosis of trisomy 13, trisomy 18, trisomy 21, Turner syndrome, triple X syndrome, XYY syndrome, Klinefelter syndrome, Di George syndrome, Angelman syndrome, Prader-Willi syndrome, or cri-du-chat syndrome.

### Application to cancer diagnosis

In one aspect, single cells are obtained from the blood of a cancer patient. Specifically, a population of bulk cells is isolated from the blood of a cancer patient. The cell population includes cancer cells consisting of peripheral circulating tumor cells (CTCs) and/or cancerous blood cells. In another aspect, the cell population includes cells obtained by biopsying a solid tumor and dispersing the biopsied tumor with a predetermined chemical and/or physical treatment. In one aspect, the cancerous blood cell is a cancerous plasma cell. In one aspect, these cancer cells in the cell population are enriched. The cell population further includes cells that are not cancer cells. In one aspect, a cell that is not cancer cell is euploid in the region on the genomic DNA to be detected. In one aspect, a cell that is not cancer cell is a normal white blood cell that circulates in the blood of a cancer patient. In one aspect, the normal white blood cell is a normal plasma cell.

In one aspect, data is provided for the diagnosis of myelodysplastic syndrome, multiple myeloma, idiopathic eosinophilia, chronic eosinophilic leukemia, acute nonlymphocytic leukemia, myeloproliferative neoplasm, chronic lymphocytic leukemia, acute myeloid leukemia, brain tumor, neuroblastoma, colon cancer, breast cancer, ovarian cancer, cervical cancer, endometrial cancer, small cell lung cancer, non-small cell lung cancer, bladder cancer, kidney cancer, liver cancer, pancreatic cancer, esophageal cancer, thyroid cancer, or head and neck cancer.

### Application example to multiple myeloma

As described in NPLs 8 and 9, multiple myeloma (MM) is a type of hematopoietic malignancy, and is an disorder in which plasma cells undergo neoplasia. Chromosomal abnormalities are found in the majority of cases of MM. Trisomy on chromosomes 3, 5, 7, 9, 11, 15, and 19 are frequently reported. Monosomy on chromosomes 13, 14, 16, and 22 is frequently found. Of these, monosomy on chromosome 13 and deletion of the long arm of chromosome 13 (13q deletion) are examples of the most frequent chromosomal mutations. These chromosomal mutations are found in approximately 50% of new patients. Of these, 85% have a monoploid chromosome in its entirety or in a portion thereof to be detected, and 15% have intra-arm deletions, including the 13q14 deletion. Biallelic deletion is rare. In addition, deletion of 17p13 on the short arm of chromosome 17 is observed in about 10%. These are one of the poor prognostic factors of MM. Another poor prognostic factor is amplification of 1q21 on the long arm of chromosome 1. It is reported to be found in 43% of untreated MM patients.

In the present embodiment, the amount of residual tumors in a patient with at least one of these chromosomal abnormalities is monitored. Specifically, the extent to which plasma cells with such a chromosomal mutation (cancerous plasma cells) remain in the body is periodically measured during the post-treatment period.

In the present embodiment, a minimal residual disease (MRD) consisting of cells with the above-mentioned chromosomal mutation is measured. Among residual diseases, MRD is particularly difficult to measure with conventional techniques. The therapeutic effect can be deeply evaluated by measuring the MRD. Based on such evaluations, physicians decide to increase, decrease, discontinue, and resume treatment.

An example of an actual inspection method is described below. In one example, bone marrow fluid or blood taken from a patient is used as a sample. Red blood cells are removed from these liquids. Hemolysis, centrifugation, and/or a chip for hydraulic classification are used to remove red blood cells.

The sample is mixed with a predetermined probe-primer set and a PCR premix, and cells are lysed. A probe-primer set that has a target within the region of 13q is selected as a set to be detected. In one aspect, a probe-primer set that has a target at a location on chromosome 18 is selected as a control.

Cluster shifts as illustrated in FIGS. 5 and 7 can detect 13q deletion on chromosome 13, namely partial haploidization. Events within a cluster have a distribution of fluorescence intensity. Combined with the necessary statistical analysis, cells with 13q deletion on chromosome 13, which are present in a range of 0 to 100% of the cell population in the sample, are detected. In one aspect, the ratio of cells, with 13q deletion on chromosome 13, to be detected is 10, 1, 0.1, 0.05, 0.04, 0.03, 0.02, or 0.01% in the sample cell population. As described above, cells with 13q deletion on chromosome 13 are considered to be cancerous plasma cells. In another aspect, a target is assigned to a region where both 13q deletion and chromosome 13 monosomy can be detected.

In one aspect, plasma cells, including circulating tumor cells, are enriched by using antibodies directed against plasma cell-specific antigens prior to measurement. As a result, cancerous plasma cells, namely tumor-forming plasma cells, present in the bone marrow fluid or blood can be detected with even higher sensitivity.

In another aspect, information on whether cells in the sample have at least one of deletion of 17p13 and amplification of 1q21 is obtained from the cluster shift. Such information helps to predict the prognosis of MM.

Application examples to myelodysplastic syndrome and other hematopoietic malignancies

Myelodysplastic syndrome (MDS) is a type of hematopoietic malignancy. At least 50% of MDS are caused by chromosomal mutations occurring in hematopoietic stem cells (specifically, aneuploidy over the entire length of a chromosome, partial aneuploidy of a chromosome, gene amplification, or gene deletion). Representative chromosomal mutations for MDS are deletion of the long arm of chromosome 5 (5q), monosomy of chromosome 7, and deletion of the long arm of chromosome 20 (20q). MRD is periodically measured by detecting cells with chromosomal mutations present in a sample.

Disorders associated with 5q deletion are independently defined as a type of MDS called 5q-syndrome. According to European and US studies, approximately 10% of MDS are 5q-syndrome. Lenalidomide (generic name) is effective for 5q-syndrome. Monitoring of the therapeutic effect of Lenalidomide is performed with the above cluster shift.

In another aspect, detection of other hematopoietic malignancies with chromosomal aneuploidies, particularly chromosomal deletions and amplifications, is performed with the above cluster shift.

### Detection of genetic mutation

In one aspect, detecting CNV detects the presence of single cells with a genetic mutation. The genetic mutation is at least one of base substitutions, base insertions, and base deletions.

In one aspect, at least one of the single cells assigned to the respective reaction compartments (reaction droplets) is an internal control that has no detectable gene mutations at least at the location of a target genome. A cluster of reaction droplets that has an amplicon amount different from that of the reaction droplet serving as an internal control is shifted from the position of the cluster without a gene mutation. These droplets in such a cluster are detected as droplets with a mutation.

In another aspect, the fluorescence intensity of an external standard is measured in advance in another well or at another time. The fluorescence intensity information of the external standard is compared with the fluorescence intensity information at the center of each cluster. Based on such comparison, the copy number of the gene mutation in each cluster is measured. The copy number of a genetic mutation can be measured even in the following case: when a cell population is dominated by cells with the genetic mutation, and thus a cell with only the wild-type gene cannot be used as an internal control.

In one aspect, the genetic mutation is introduced into an unspecified region of a genome by a lentiviral vector or another vector. Examples of cells with vector-induced genetic mutations are chimeric antigen receptor T cells (CAR-T cells) and TCR-T cells. For the detection in the present embodiment, the target may be DNA introduced by a vector. For example, the copy number of DNA introduced by a vector may be detected.

### Test example 8: Copy number detection in human specimens

Peripheral blood of a healthy subject was hemolyzed and added to the PCR premix to prepare a PCR reaction solution. The above PCR reaction solution was made into droplets by using QX200 Droplet Generator (BioRad Laboratories, Inc.), and cells were lysed in the respective droplets. Subsequently, PCR was performed under general conditions within each droplet. The number of PCR cycles was 23. The PCR reaction was in the exponential amplification phase.

In the present test example, 23 types of probes each including a fluorescent material (FAM) with the first fluorescence wavelength were assigned to chromosome 13. In addition, 25 types of probes each including a fluorescent material (HEX) with the second fluorescence wavelength were assigned to chromosome 18. The probes were mixed together in one droplet.

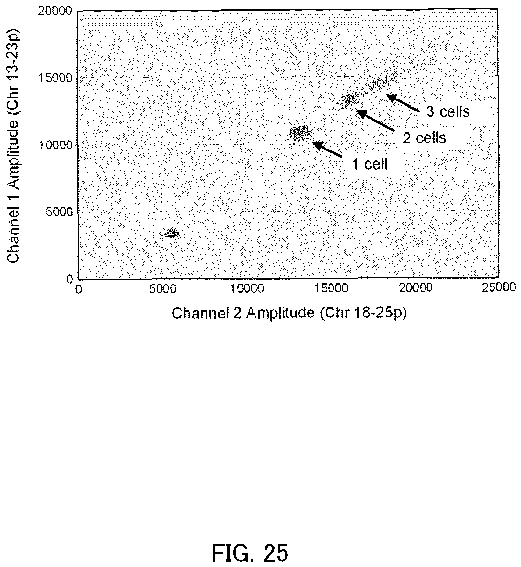

FIG. 25 illustrate the results of single cell-droplet PCR performed on human blood. The cluster in the center is the measurement result corresponding to droplets each containing one cell. The cluster above and to the right of the cluster in the center is the measurement result corresponding to droplets each containing two cells. As shown in the drawing, the clusters can be clearly distinguished from each other, suggesting that the copy number of a nucleic acid can be detected also when human blood is used as a specimen.

Next, peripheral blood of a healthy subject was hemolyzed, and CD45-positive cells were isolated by using EasySep Human CD45 Depletion Kit II (STEMCELL Technologies). The resulting CD45 cells were added to the PCR premix to prepare a PCR reaction solution. The above PCR reaction solution was made into droplets by using QX200 Droplet Generator (BioRad Laboratories, Inc.), and cells were lysed in the respective droplets. Subsequently, PCR was performed under general conditions within each droplet. The number of PCR cycles was 23. The PCR reaction was in the exponential amplification phase.

Also in the present test example, 23 types of probes each including a fluorescent material (FAM) with the first fluorescence wavelength were assigned to chromosome 13. In addition, 25 types of probes each including a fluorescent material (HEX) with the second fluorescence wavelength were assigned to chromosome 18. The probes were mixed together in one droplet.

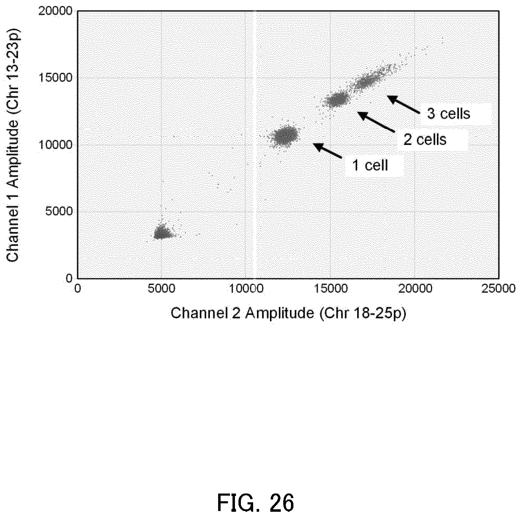

FIG. 26 illustrate the results of single cell-droplet PCR performed on CD45 cells derived from human blood. The cluster in the center is the measurement result corresponding to droplets each containing one cell. The cluster above and to the right of the cluster in the center is the measurement result corresponding to droplets each containing two cells. As shown in the drawing, the clusters can be clearly distinguished from each other, suggesting that the copy number of a nucleic acid can be detected also when cells were enriched with a specific antigen as a target.

Next, a cell population obtained by mixing the CD45-positive cells and cells of the human cultured cell line GM13721 at a predetermined ratio was added to the PCR premix to prepare a PCR reaction solution. The above PCR reaction solution was made into droplets by using QX200 Droplet Generator (BioRad Laboratories, Inc.), and cells were lysed in the respective droplets. Subsequently, PCR was performed under general conditions within each droplet. The number of PCR cycles was 23. The PCR reaction was in the exponential amplification phase. In the CD45-positive cell, chromosomes 13 and 18 are euploid, that is, diploid. In the human cultured cell line GM13721, a partial region of one long arm of chromosome 13 is monoploid, and chromosome 18 is euploid.

Also in the present test example, 23 types of probes each including a fluorescent material (FAM) with the first fluorescence wavelength were assigned to the defective region of chromosome 13. In addition, 25 types of probes each including a fluorescent material (HEX) with the second fluorescence wavelength were assigned to chromosome 18. The probes were mixed together in one droplet.

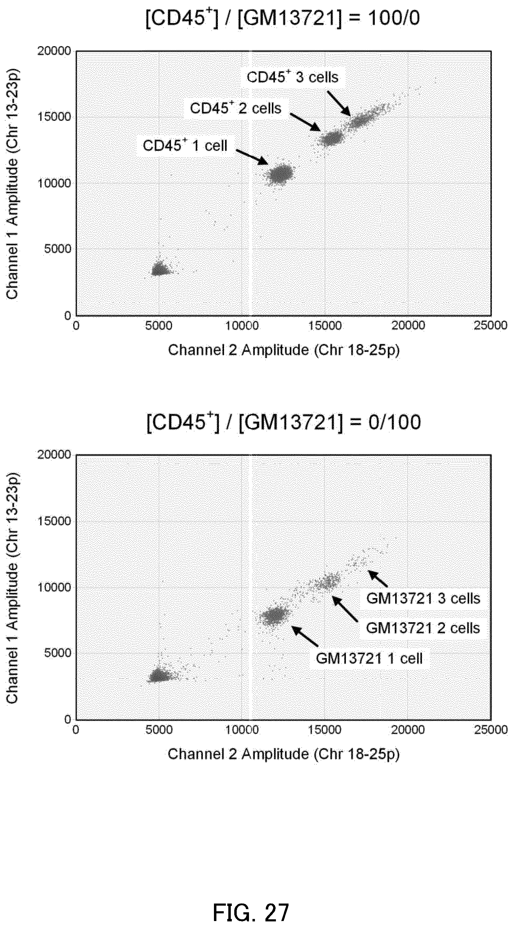

FIG. 27 illustrates scatters of droplets in homozygous cell populations. The upper part of FIG. 27 illustrates the results of single cell-droplet PCR performed on the CD45-positive cells. The lower part of FIG. 27 illustrates the results of single cell-droplet PCR performed on the human cultured cell line GM13721.

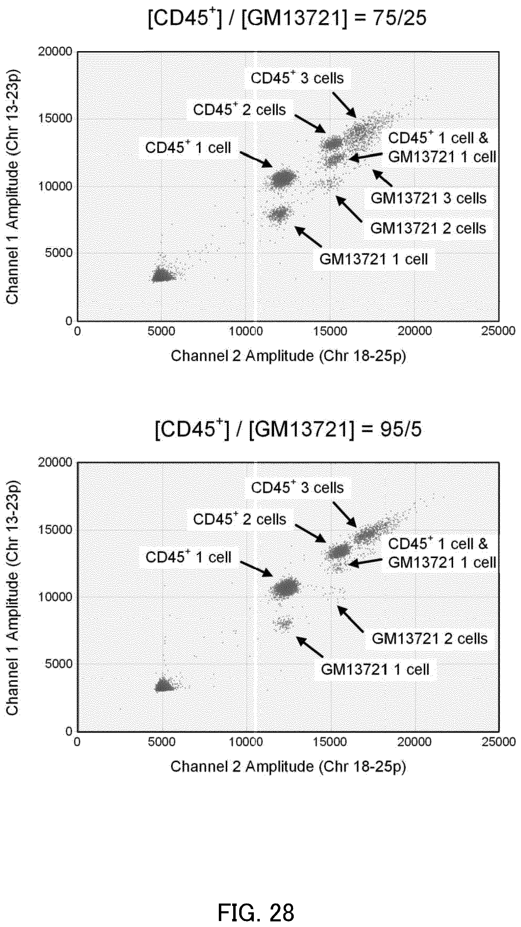

FIG. 28 illustrates scatters of droplets in heterogeneous cell populations. The upper part of FIG. 28 illustrates the results of single cell-droplet PCR performed on a cell population in which the mixing ratio of the CD45-positive cells and the human cultured cell line GM13721 is 75/25. The lower part of FIG. 28 illustrates the results of single cell-droplet PCR performed on a cell population in which the mixing ratio of the CD45-positive cells and the human cultured cell line GM13721 is 95/5.

These results show that the CD45-positive cells and the human cultured cell line GM13721 are clearly distinguished from each other. This suggests that cells with abnormal copy numbers can also be detected from a cell population derived from human blood.

### Test example 9: Evaluation of detection sensitivity when using human specimens

A cell population in which the CD45-positive cells and cells of the human cultured cell line GM13721 were mixed at a predetermined ratio was prepared in the same manner as in Test Example 8 above. The proportion of cells of the line GM13721 in the cell population was 0%, 0.1%, or 3%. Each cell population was added to the PCR premix to prepare a PCR reaction solution. The above PCR reaction solution was made into droplets by using QX200 Droplet Generator (BioRad Laboratories, Inc.), and cells were lysed in the respective droplets. Subsequently, PCR was performed under general conditions within each droplet. The number of PCR cycles was 23. The PCR reaction was in the exponential amplification phase. In the CD45-positive cell, chromosomes 13 and 18 are euploid, that is, diploid. In the human cultured cell line GM13721, a partial region of one long arm of chromosome 13 is monoploid, and chromosome 18 is euploid.

Also in the present test example, 23 types of probes each including a fluorescent material (FAM) with the first fluorescence wavelength were assigned to the defective region of chromosome 13. In addition, 25 types of probes each including a fluorescent material (HEX) with the second fluorescence wavelength were assigned to chromosome 18. The probes were mixed together in one droplet.

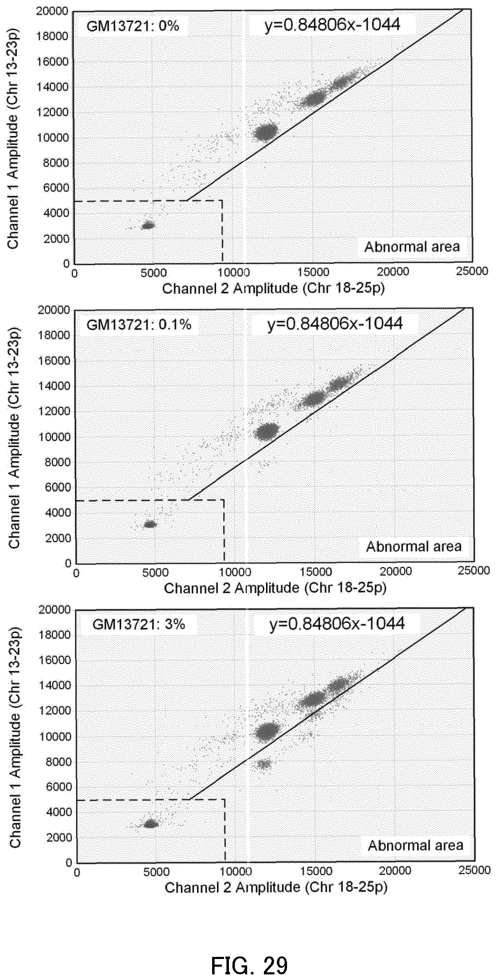

FIG. 29 illustrates scatters of droplets for respective cell populations. The upper part of FIG. 29 illustrates the results of single cell-droplet PCR performed on a cell population with the cell proportion of the line GM13721 of 0%. The middle part of FIG. 29 illustrates the results of single cell-droplet PCR performed on a cell population with the cell proportion of the line GM13721 of 0.1%. The lower part of FIG. 29 illustrates the results of single cell-droplet PCR performed on a cell population with the cell proportion of the line GM13721 of 3%.

In the present test example, a dot having fluorescence intensity of 5,000 or more at the first fluorescence wavelength (FAM) or fluorescence intensity of 9,000 or more at the second fluorescence wavelength (HEX) was used as a positive droplet to be evaluated. Using the cutoff (y = 0.84806x-1044) determined by the same manner as in Test Example 7, the ratio of abnormal droplets (droplets that is considered to include monoploid chromosome 13) with respect to the positive droplets was calculated.

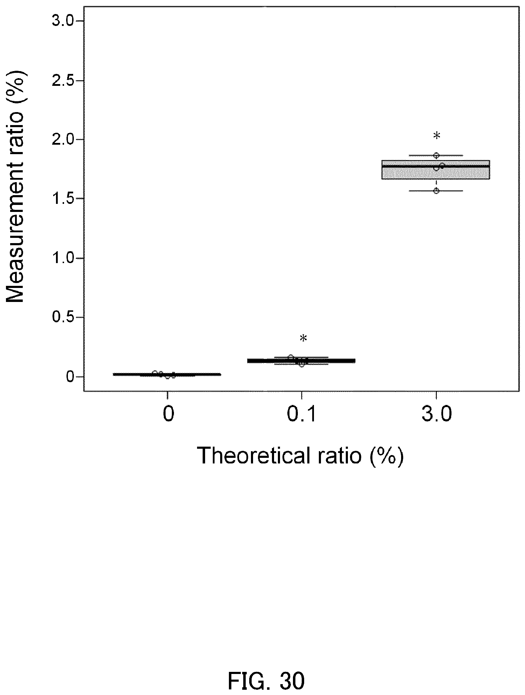

FIG. 30 illustrates a boxplot showing the detection results of abnormal droplets for each cell population. Asterisks indicate p < 0.05 (Steel test results compared respectively to control (0%) after confirming that the results by Kruskal-Wallis test were significant). The graph shows that the detection results for the cell populations with the cell proportion of the line GM13721 of 3% or 0.1% are significantly different from the detection results for the cell population with the cell proportion of the line GM13721 of 0%. Therefore, it can be seen that at least 0.1% can be detected.

### Test example 10: Detection of copy number in reverse transcription product of RNA

Using a reverse transcription product of RNA in a single cell as DNA sample, the copy number of mRNA for the B2M gene and the copy number for the mRNA for the GAPDH gene were detected.

In the present test example, single cell-droplet RT-PCR was performed on the human cultured cell line GM22948. The premix contains primers for the B2M gene, primers for the GAPDH gene, probes for the B2M gene (FAM label), probes for the GAPDH gene (HEX label), and reverse transcriptase (SuperScript IV). The primers for both genes were designed to flank an intron in order to amplify the mRNA of the genes by PCR, rather than the genes in the genomic DNA.

Specifically, a PCR reaction solution was prepared by adding 10,000 cells (line GM22948) and 2 µL of a cell suspension containing PBS to the PCR premix containing sodium dodecyl sulfate. The concentrations of primers, probes, and the like in the PCR reaction solution are as follows.
- 1X SuperScript IV (Thermo Fisher Scientific)
- 1X ddPCR Multiplex Super Mix (BioRad Laboratories, Inc.)
- 200 nM B2M-FAM probe
- 400 nM B2M primer
- 200 nM GAPDH-HEX probe
- 400 nM GAPDH primer
- Distilled water (for filling up)

The above PCR reaction solution was made into droplets by using QX200 Droplet Generator (BioRad Laboratories, Inc.), the cells were lysed in each droplet, and the cells were allowed to stand at 50°C for 10 minutes for reverse transcription. After denaturing the DNA at 95°C for 10 minutes, PCR was performed under general conditions. The number of PCR cycles was 23. The PCR reaction was in the exponential amplification phase.

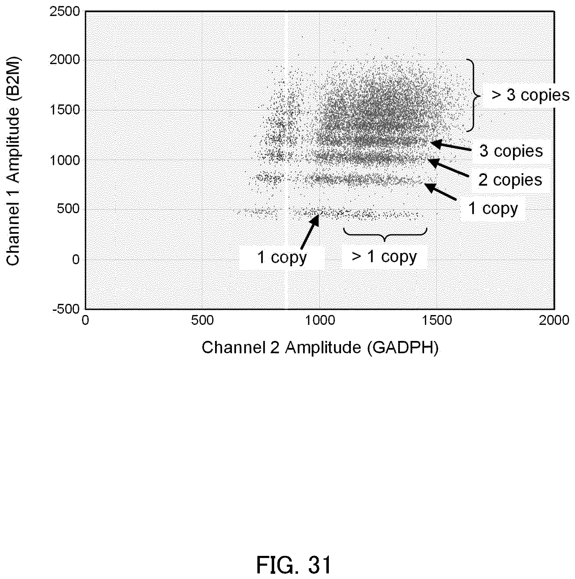

FIG. 31 illustrates the results of single cell-droplet RT-PCR performed on the human cultured cell line GM22948. This result shows that the copy number of RNA can be detected also when the reverse transcription product of RNA is used as the DNA sample.

No positive droplets were detected when the premix did not contain reverse transcriptase. That is, with the primers designed to flank the intron as described above, both genes in the genomic DNA were hardly amplified.

### Test Example 11: Detection of copy number of mitochondrial DNA

Using mitochondrial DNA as a DNA sample, the copy number of the ND1 gene was detected.

In the present test example, single cell-droplet PCR was performed on the human cultured cell line GM22948. Each droplet contains genomic DNA as well as mitochondrial DNA within a single cell. The premix contains primers for chromosome 13 (23 types), primers for ND1 gene of mitochondrial DNA, probes for chromosome 13 (23 types, FAM-labeled), and probes for the ND1 gene (HEX-labeled).

Specifically, a PCR reaction solution was prepared by adding 10,000 cells (line GM22948) and 2 µL of a cell suspension containing PBS to the PCR premix containing sodium dodecyl sulfate. The concentrations of primers, probes, and the like in the PCR reaction solution are as follows.
- 1X ddPCR Multiplex Super Mix (BioRad Laboratories, Inc.)
- 25 nM Chromosome 13-FAM probe
- 1 µM Chromosome 13 primer
- 500 nM ND1-HEX probe
- 1 µM ND1 primer
- Distilled water (for filling up)

The above PCR reaction solution was made into droplets by using QX200 Droplet Generator (BioRad Laboratories, Inc.), and the cells were lysed in each droplet. After denaturing the DNA at 95°C for 10 minutes, PCR was performed under general conditions. The number of PCR cycles was 23. The PCR reaction was in the exponential amplification phase.

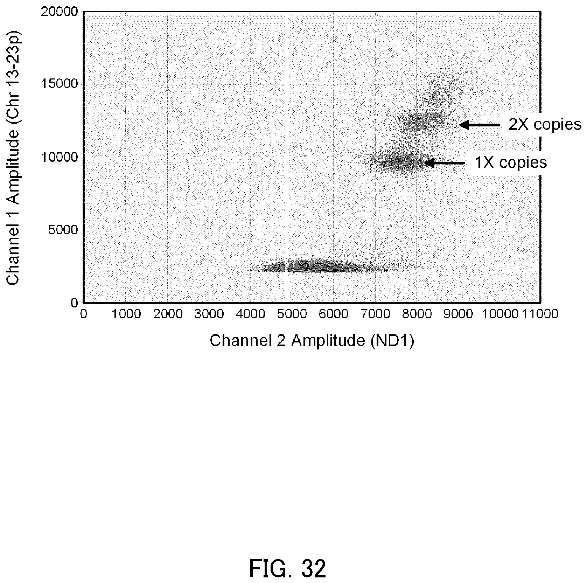

FIG. 32 illustrates the results of single cell-droplet PCR performed on the human cultured cell line GM22948. The cluster slightly right in the center is the measurement result corresponding to droplets each containing one cell (containing about 1X mitochondrial DNA). The cluster above and to the right of the cluster in the center is the measurement result corresponding to droplets each containing two cells (containing about 2X mitochondrial DNA). The clusters are shifted to the right as the copy number of mitochondrial DNA increased. This result shows that the copy number of genes in mitochondrial DNA can be detected also when mitochondrial DNA is used as the DNA sample.

### Use of mitochondrial DNA as control

To detect CNVs in the target assigned regions, a control is assigned to a region considered to have a small CNV and be euploid. For cells with frequent chromosomal abnormalities, such as cancer cells, it is possible that a suitable chromosome cannot be found as a control. In such cases, mitochondrial DNA may be used as a control. That is, one of the fluorescence wavelengths to be used is selected, and a probe including a fluorescent material with the selected fluorescence wavelength is assigned to mitochondrial DNA.

For example, DNA sample contains a set of genomic DNA in a single cell and mitochondrial DNA in the single cell. The PCR reaction system includes a probe (including a fluorescent material with a first fluorescence wavelength) assigned to a region on genomic DNA and a probe (including a fluorescent material with a second fluorescence wavelength) assigned to mitochondrial DNA. The region includes one or more targets. In the reaction compartment, targets contained in the set of genomic DNA and in the mitochondrial DNA are amplified by PCR. Amplicons of the genomic DNA and mitochondrial DNA are quantified in each reaction compartment in a cycle during which amplification of the target contained in the mitochondrial DNA reaches a plateau and amplification of the target contained in the genomic DNA reaches an exponential amplification phase. At this time, fluorescence of the first and second fluorescence wavelengths is detected from one reaction compartment. This configuration can distinguish a reaction compartment, in which the target contained in the genomic DNA and the target contained in the mitochondrial DNA are amplified, from a reaction compartment, in which only a contaminating cell-free nucleic acid is amplified.

During droplet PCR, amplification using mitochondrial DNA as a template proceeds fast. Therefore, primers or probes are depleted early. This is because the copy number of mitochondrial DNA per cell is larger than the copy number of genomic DNA per cell. Therefore, the PCR is stopped at a cycle number where the reaction for mitochondrial DNA reaches a plateau. However, at such a cycle number, the amplification using the genomic DNA of the chromosome as a template is in the exponential amplification phase.

For example, in FIGS. 4 to 7, when the probe of the second fluorescence wavelength (2nd color) is assigned to a chromosome with a large CNV, the intensity of the second fluorescence wavelength is less likely to converge to form a cluster. In other words, the intensity of the second fluorescence wavelength does not converge to an intensity equivalent to euploid.

For example, in FIGS. 4 to 7, when the second fluorescence wavelength is assigned to mitochondrial DNA, the fluorescence intensity reaches a plateau in the PCR at the second fluorescence wavelength. Therefore, the intensity of the second fluorescence wavelength converges to form a cluster.

### Industrial Applicability

The detection method of the present invention is particularly advantageous for, for example, prenatal diagnosis and cancer diagnosis.

### Reference Signs List

17 Cell population
19 Oil
20 Droplet
21 Set of genomic DNA
22a, 22b Droplet
31 Premix
33a, 33b Droplet
35a, 35b Probe
39 Cluster
40a, 40b, 40c Cluster
41a, 41b, 41c Cluster
44a, 44b Cluster

## Claims

1. A method for detecting a copy number of a specific nucleic acid per single cell in a cell population, the method comprising:
amplifying, in a reaction compartment of a plurality of reaction compartments each containing a Polymerase Chain Reaction (PCR) system and a DNA sample that is derived from a nucleic acid in a single cell, a target contained in the DNA sample by PCR; and
quantifying the copy number of the specific nucleic acid by quantifying an amplicon obtained by the PCR, the quantifying the amplicon being performed by measuring an intensity of fluorescence in each of the plurality of reaction compartments during an exponential amplification phase; wherein
the DNA sample is a set of genomic DNA in the single cell, a reverse transcription product of RNA in the single cell, or mitochondrial DNA in the single cell;
the amplicon is quantified by a fluorescent probe method;
**characterized in that**
the PCR reaction system contains a plurality of probes respectively including fluorescent materials with fluorescence wavelengths different from each other, the plurality of probes being respectively assigned to regions different from each other on the DNA sample;
each of the regions contains the target or a plurality of the targets; and
in the quantifying the amplicon, by measuring an intensity of fluorescence of a plurality of wavelengths from each of the plurality of reaction compartments, the reaction compartment in which the target or the plurality of targets contained in the DNA sample are amplified is distinguished from the reaction compartment in which only a contaminating cell-free nucleic acid is amplified.

2. The method according to claim 1, wherein
the quantifying the amplicon includes
stopping a cycle of the PCR during the exponential amplification phase and measuring the intensity of the fluorescence in each of the plurality of reaction compartments, and
quantifying the amplicon obtained by the PCR from the intensity of the fluorescence.

3. The method according to claim 1, wherein:
each of the regions contains 5 to 100 of the targets to which a plurality of probes including fluorescent materials with fluorescence wavelengths identical to each other are assigned, respectively; and
in the quantifying the amplicon, for each of the regions, the amplicon is quantified by collectively measuring an intensity of fluorescence from the plurality of probes, regardless of a difference between the targets.

4. The method according to any one of claims 1 to 3, wherein:
the DNA sample contains a set of genomic DNA and mitochondrial DNA both in the single cell;
the PCR reaction system contains a first probe including a fluorescent material with a first fluorescence wavelength and a second probe including a fluorescent material with a second fluorescence wavelength, the first probe being assigned to a region on the genomic DNA, the second probe being assigned to the mitochondrial DNA;
the region contains the target or the plurality of targets;
in the amplifying by the PCR, in each of the plurality of reaction compartments, the target or the plurality of targets contained in the set of genomic DNA and a target contained in the mitochondrial DNA are amplified by the PCR;
in the quantifying the amplicon, in each of the plurality of reaction compartments, the amplicon of the genomic DNA and the amplicon of mitochondrial DNA are quantified in a cycle during which amplification of the target contained in the mitochondrial DNA reaches a plateau and amplification of the target or the plurality of targets contained in the genomic DNA reaches the exponential amplification phase; and
in the quantifying the amplicon, by measuring an intensity of fluorescence of the first fluorescence wavelength and the second fluorescence wavelength from each of the plurality of reaction compartments, the reaction compartment in which the target or the plurality of targets contained in the genomic DNA and the target contained in the mitochondrial DNA are amplified is distinguished from the reaction compartment in which only a contaminating cell-free nucleic acid is amplified.

5. The method according to any one of claims 1 to 4, further comprising, generating the reaction compartment by lysing the single cell in a compartment containing the single cell, a cell lysis reagent, and a PCR premix.

6. The method according to any one of claims 1 to 5, further comprising:
lysing the single cell in a compartment containing the single cell; and
generating the reaction compartment by combining the compartment containing the single cell lysed with a compartment containing a PCR premix.

7. The method according to any one of claims 1 to 6, further comprising:
mixing a population of cell nuclei and a PCR premix in bulk; and
generating a plurality of the reaction compartments by separating the cell nuclei in the population of the cell nuclei together with the PCR premix from each other.

8. The method according to any one of claims 1 to 7, wherein the reaction compartment is a reaction droplet that is a droplet containing the DNA sample and the PCR reaction system.

9. The method according to any one of claims 1 to 8, wherein:
in the quantifying the copy number of the specific nucleic acid, the copy number of the specific nucleic acid is quantified by using a cutoff value as an external standard; and
the cutoff value is set in advance based on a result of the quantifying the amplicon obtained by the PCR for a cell in which the copy number of the specific nucleic acid is known.

10. The method according to claim 9, wherein before the result of the quantifying the amplicon obtained by the PCR is used to set the cutoff value, the result is corrected by using a result of a quantifying for a negative cluster.

11. The method according to any one of claims 1 to 10, wherein:
the DNA sample contains a set of genomic DNA in the single cell; and
the method further comprises detecting, from a result of the quantifying the amplicon, a presence of the single cell with at least one member selected from the group consisting of aneuploidy over an entire length of a chromosome, partial aneuploidy of a chromosome, gene amplification, and gene deletion.

12. The method according to claim 11, wherein the single cell contains the genomic DNA at least with a chromosomal mutation selected from below:
- aneuploidy of chromosome 21,
- aneuploidy of chromosome 18,
- aneuploidy of chromosome 13,
- aneuploidy of Y chromosome,
- aneuploidy of X chromosome,
- deletion of the 22q11.2 region on a long arm of chromosome 22,
- deletion of the 5q region on a short arm of chromosome 5,
- deletion of the 15q11-q13 region on a long arm of chromosome 15,
- amplification of a long arm of chromosome 1,
- deletion of a short arm of chromosome 17,
- deletion of a long arm of chromosome 13,
- deletion of a long arm of chromosome 4,
- deletion of a long arm of chromosome 5,
- deletion of a long arm of chromosome 7,
- amplification of chromosome 8,
- deletion of chromosome 11,
- aneuploidy of chromosome 12,
- deletion of a long arm of chromosome 20,
- deletion of a long arm of chromosome 19,
- deletion of chromosome 1,
- deletion of a long arm of chromosome 18,
- deletion of a short arm of chromosome 8,
- deletion of chromosome 4,
- amplification of a long arm of chromosome 8,
- deletion of a long arm of chromosome 16,
- amplification of a short arm of chromosome 5,
- amplification of a long arm of chromosome 3,
- deletion of a short arm of chromosome 3,
- deletion of a short arm of chromosome 9,
- gene amplification of MYCN gene,
- gene amplification of HER2 gene, and
- gene amplification of MET gene.

13. The method according to claim 11, further comprising:
generating data that includes information on whether or not the presence of the single cell is detected, wherein
the cell population is isolated from amniotic fluid or maternal blood so as to contain a fetal cell; and
the data is provided for a diagnosis of trisomy 13, trisomy 18, trisomy 21, Turner syndrome, triple X syndrome, XYY syndrome, Klinefelter syndrome, Di George syndrome, Angelman syndrome, Prader-Willi syndrome, or cri-du-chat syndrome.

14. The method according to claim 11, further comprising:
generating data that includes information on whether or not the presence of the single cell is detected, wherein
the cell population is isolated from a patient, and
the data is provided for a diagnosis of myelodysplastic syndrome, multiple myeloma, idiopathic eosinophilia, chronic eosinophilic leukemia, acute nonlymphocytic leukemia, myeloproliferative neoplasm, chronic lymphocytic leukemia, acute myeloid leukemia, brain tumor, neuroblastoma, colon cancer, breast cancer, ovarian cancer, cervical cancer, endometrial cancer, small cell lung cancer, non-small cell lung cancer, bladder cancer, kidney cancer, liver cancer, pancreatic cancer, esophageal cancer, thyroid cancer, or head and neck cancer.

## Patentansprüche

1. Verfahren zum Detektieren einer Kopienzahl einer spezifischen Nukleinsäure pro Einzelzelle in einer Zellpopulation, wobei das Verfahren umfasst:
Amplifizieren eines in der DNA-Probe enthaltenen Ziels durch PCR in einem Reaktionskompartiment aus einer Vielzahl von Reaktionskompartimenten, die jeweils ein Polymerase-Kettenreaktionssystem (PCR-System) und eine DNA-Probe enthalten, die von einer Nukleinsäure in einer einzelnen Zelle stammt; und
Quantifizieren der Kopienzahl der spezifischen Nukleinsäure durch Quantifizieren eines durch die PCR erhaltenen Amplikons, wobei das Quantifizieren des Amplikons durch Messen einer Fluoreszenzintensität in jedem der mehreren Reaktionskammern während einer exponentiellen Amplifikationsphase durchgeführt wird; wobei
die DNA-Probe ein Satz genomischer DNA in der einzelnen Zelle, ein reverses Transkriptionsprodukt von RNA in der einzelnen Zelle oder mitochondriale DNA in der einzelnen Zelle ist;
das Amplikon durch ein Fluoreszenzsondenverfahren quantifiziert wird;
**dadurch gekennzeichnet, dass**
das PCR-Reaktionssystem mehrere Sonden enthält, die jeweils Fluoreszenzmaterialien mit voneinander unterschiedlichen Fluoreszenzwellenlängen umfassen, wobei die mehreren Sonden jeweils voneinander unterschiedlichen Regionen auf der DNA-Probe zugeordnet sind;
jeder der Bereiche das Ziel oder mehrere der Ziele enthält; und
bei der Quantifizierung des Amplikons durch Messen einer Fluoreszenzintensität einer Vielzahl von Wellenlängen aus jedem der Vielzahl von Reaktionskompartimenten, das Reaktionskompartiment, in dem das Ziel oder die Vielzahl von Zielen, die in der DNA Probe enthalten sind, unterschieden werden, von dem Reaktionskompartiment, in dem nur eine von kontaminierenden Zellen freie Nukleinsäure amplifiziert wird.

2. Verfahren gemäß Anspruch 1, wobei
die Quantifizierung des Amplikons umfasst
das Anhalten eines Zyklus der PCR während der exponentiellen Amplifikationsphase und das Messen der Intensität der Fluoreszenz in jedem der mehreren Reaktionskompartimente, und
die Quantifizierung des durch die PCR erhaltenen Amplikons anhand der Intensität der Fluoreszenz.

3. Verfahren gemäß Anspruch 1, wobei
jede der Regionen 5 bis 100 der Ziele enthält, denen jeweils mehrere Sonden zugeordnet sind, die fluoreszierende Materialien mit identischen Fluoreszenzwellenlängen enthalten; und
bei der Quantifizierung des Amplikons für jede der Regionen das Amplikon durch gemeinsames Messen einer Fluoreszenzintensität aus der Vielzahl von Sonden quantifiziert wird, unabhängig von einem Unterschied zwischen den Targets.

4. Verfahren gemäß einem der Ansprüche 1 bis 3, wobei
die DNA-Probe einen Satz genomischer DNA und mitochondrialer DNA in der gleichen einzelnen Zelle enthält;
das PCR-Reaktionssystem eine erste Sonde mit einem fluoreszierenden Material mit einer ersten Fluoreszenzwellenlänge und eine zweite Sonde mit einem fluoreszierenden Material mit einer zweiten Fluoreszenzwellenlänge enthält, wobei die erste Sonde einer Region auf der genomischen DNA zugeordnet ist und die zweite Sonde der mitochondrialen DNA zugeordnet ist;
die Region das Ziel oder die Vielzahl von Zielen enthält;
beim Amplifizieren durch die PCR in jedem der mehreren Reaktionskompartimente das Ziel oder die mehreren Ziele, die in dem Satz genomischer DNA enthalten sind, und ein Ziel, das in der mitochondrialen DNA enthalten ist, durch die PCR amplifiziert;
bei der Quantifizierung des Amplikons in jedem der mehreren Reaktionskompartimente das Amplikon der genomischen DNA und das Amplikon der mitochondrialen DNA in einem Zyklus quantifiziert werden, in dem das Amplifizieren des in der mitochondrialen DNA enthaltenen Ziels ein Plateau erreicht und das Amplifiziren des in der genomischen DNA enthaltenen Ziels oder der mehreren Ziele die exponentielle Amplifizierphase erreicht; und
bei der Quantifizierung des Amplikons durch Messen einer Fluoreszenzintensität der ersten Fluoreszenzwellenlänge und der zweiten Fluoreszenzwellenlänge aus jedem der mehreren Reaktionskompartimente das Reaktionskompartiment, in dem das Zielmolekül oder die mehreren Zielmoleküle, die in der genomischen DNA enthalten sind, und das Zielmolekül, das in der mitochondrialen DNA enthalten ist, amplifiziert werden, von dem Reaktionskompartiment unterschieden wird, in dem nur eine kontaminierende zellfreie Nukleinsäure amplifiziert wird.

5. Verfahren gemäß einem der Ansprüche 1 bis 4, das ferner umfasst: Erzeugen des Reaktionskompartiments durch Lysieren der einzelnen Zelle in einem Kompartiment, das die einzelne Zelle, ein Zelllyse-Reagenz und eine PCR-Vormischung enthält.

6. Verfahren gemäß einem der Ansprüche 1 bis 5, das ferner umfasst:
Lysieren der einzelnen Zelle in einem Kompartiment, in dem die einzelne Zelle enthalten ist; und
Erzeugen des Reaktionskompartiments durch Kombinieren des Kompartiments, das die lysierte Einzelzelle enthält, mit einem Kompartiment, das eine PCR-Vormischung enthält.

7. Verfahren gemäß einem der Ansprüche 1 bis 6, das ferner umfasst:
Mischen einer Population von Zellkernen und einer PCR-Vormischung in einer Masse bzw. In-Bulk; und
Erzeugen einer Vielzahl von Reaktionskompartimenten durch Trennen der Zellkerne in der Population der Zellkerne zusammen mit dem PCR-Premix voneinander.

8. Verfahren gemäß einem der Ansprüche 1 bis 7, wobei das Reaktionskompartiment ein Reaktions-Tröpfchen ist, das die DNA-Probe und das PCR-Reaktionssystem enthält.

9. Verfahren gemäß einem der Ansprüche 1 bis 8, wobei
bei der Quantifizierung der Kopienzahl der spezifischen Nukleinsäure die Kopienzahl der spezifischen Nukleinsäure unter Verwendung eines Cutoff-Wertes als externen Standard quantifiziert wird; und
der Cutoff-Wert im Voraus auf der Grundlage eines Ergebnisses der Quantifizierung des durch die PCR erhaltenen Amplikons für eine Zelle festgelegt wird, in der die Kopienzahl der spezifischen Nukleinsäure bekannt ist.

10. Verfahren gemäß Anspruch 9, wobei vor der Verwendung des Ergebnisses der Quantifizierung des durch die PCR erhaltenen Amplikons zur Festlegung des Cutoff-Wertes das Ergebnis unter Verwendung eines Ergebnisses einer Quantifizierung für einen negativen Cluster korrigiert wird.

11. Verfahren gemäß einem der Ansprüche 1 bis 10, wobei:
die DNA-Probe einen Satz genomischer DNA in der einzelnen Zelle enthält; und
das Verfahren ferner umfasst, dass aus einem Ergebnis der Quantifizierung des Amplikons das Detektieren des Vorhandenseins der einzelnen Zelle mit mindestens einem Mitglied, ausgewählt aus der Gruppe bestehend aus Aneuploidie über die gesamte Länge eines Chromosoms, partieller Aneuploidie eines Chromosoms, Genamplifikation und Gendeletion, durchgeführt wird.

12. Verfahren gemäß Anspruch 11, wobei die einzelne Zelle die genomische DNA mindestens mit einer chromosomalen Mutation enthält, die aus den folgenden ausgewählt ist:
- Aneuploidie von Chromosom 21,
- Aneuploidie von Chromosom 18,
- Aneuploidie von Chromosom 13,
- Aneuploidie des Y-Chromosoms,
- Aneuploidie des X-Chromosoms,
- Deletion der Region 22q11.2 auf dem langen Arm von Chromosom 22,
- Deletion der Region 5q auf dem kurzen Arm von Chromosom 5,
- Deletion der Region 15q11-q13 auf dem langen Arm von Chromosom 15,
- Amplifikation bzw. Amplifitieren eines langen Arms von Chromosom 1,
- Deletion eines kurzen Arms von Chromosom 17,
- Deletion eines langen Arms von Chromosom 13,
- Deletion eines langen Arms von Chromosom 4,
- Deletion eines langen Arms von Chromosom 5,
- Deletion eines langen Arms von Chromosom 7,
- Amplifikation von Chromosom 8,
- Deletion von Chromosom 11,
- Aneuploidie von Chromosom 12,
- Deletion eines langen Arms von Chromosom 20,
- Deletion eines langen Arms von Chromosom 19,
- Deletion von Chromosom 1,
- Deletion eines langen Arms von Chromosom 18,
- Deletion eines kurzen Arms von Chromosom 8,
- Deletion von Chromosom 4,
- Amplifikation eines langen Arms von Chromosom 8,
- Deletion eines langen Arms von Chromosom 16,
- Amplifikation eines kurzen Arms von Chromosom 5,
- Amplifikation eines langen Arms von Chromosom 3,
- Deletion eines kurzen Arms von Chromosom 3,
- Deletion eines kurzen Arms von Chromosom 9,
- Genamplifikation des MYCN-Gens,
- Genamplifikation des HER2-Gens und
- Genamplifikation des MET-Gens.

13. Verfahren gemäß Anspruch 11, das ferner umfasst:
Erzeugen von Daten, die Informationen darüber enthalten, ob das Vorhandensein der einzelnen Zelle detektiert wird oder nicht, wobei
die Zellpopulation aus Fruchtwasser oder mütterlichem Blut isoliert wird, so dass sie eine fötale Zelle enthält; und
die Daten für eine Diagnose von Trisomie 13, Trisomie 18, Trisomie 21, Turner-Syndrom, Triple-X-Syndrom, XYY-Syndrom, Klinefelter-Syndrom, Di-George-Syndrom, Angelman-Syndrom, Prader-Willi-Syndrom oder Cri-du-chat-Syndrom bereitgestellt werden.

14. Verfahren gemäß Anspruch 11, das ferner umfasst:
Erzeugen von Daten, die Informationen darüber enthalten, ob das Vorhandensein der einzelnen Zelle detektiert wurde oder nicht, wobei
die Zellpopulation von einem Patienten isoliert wird und
die Daten für eine Diagnose von myelodysplastischem Syndrom, multiplem Myelom, idiopathischer Eosinophilie, chronischer eosinophiler Leukämie, akuter nichtlymphozytischer Leukämie, myeloproliferativer Neoplasie, chronischer lymphozytischer Leukämie, akuter myeloischer Leukämie, Hirntumor, Neuroblastom, Darmkrebs, Brustkrebs, Eierstockkrebs, Gebärmutterhalskrebs, Gebärmutterschleimhautkrebs, kleinzelligem Lungenkrebs, nicht-kleinzelligem Lungenkrebs, Blasenkrebs, Nierenkrebs, Leberkrebs, Bauchspeicheldrüsenkrebs, Speiseröhrenkrebs, Schilddrüsenkrebs oder Kopf-Hals-Krebs bereitgestellt werden.

## Revendications

1. Procédé pour détecter un nombre de copies d'un acide nucléique spécifique par cellule unique dans une population cellulaire, le procédé comprenant :
l'amplification, dans un compartiment de réaction d'une pluralité de compartiments de réaction contenant chacun un système de réaction en chaîne par polymérase (PCR) et un échantillon d'ADN qui est dérivé d'un acide nucléique dans une cellule unique, d'une cible contenue dans l'échantillon d'ADN par PCR ; et
la quantification du nombre de copies de l'acide nucléique spécifique en quantifiant un amplicon obtenu par la PCR, la quantification de l'amplicon étant effectuée en mesurant une intensité de fluorescence dans chacun de la pluralité de compartiments de réaction pendant une phase d'amplification exponentielle ; dans lequel
l'échantillon d'ADN est un ensemble d'ADN génomique dans la cellule unique, un produit de transcription inverse d'ARN dans la cellule unique ou de l'ADN mitochondrial dans la cellule unique ;
l'amplicon est quantifié par un procédé par sonde fluorescente ;
**caractérisé en ce que**
le système réactionnel PCR contient une pluralité de sondes incluant respectivement des matériaux fluorescents présentant des longueurs d'onde de fluorescence différentes les unes des autres, la pluralité de sondes étant respectivement affectées à des régions différentes les unes des autres sur l'échantillon d'ADN ;
chacune des régions contient la cible ou une pluralité des cibles ; et
lors de la quantification de l'amplicon, en mesurant une intensité de fluorescence d'une pluralité de longueurs d'onde à partir de chacun de la pluralité de compartiments de réaction, le compartiment de réaction dans lequel la cible ou la pluralité de cibles contenues dans l'échantillon d'ADN sont amplifiées est différencié du compartiment de réaction dans lequel seul un acide nucléique acellulaire contaminant est amplifié.

2. Procédé selon la revendication 1, dans lequel
la quantification de l'amplicon inclut
l'arrêt d'un cycle de la PCR pendant la phase d'amplification exponentielle et la mesure de l'intensité de la fluorescence dans chacun de la pluralité de compartiments de réaction, et
la quantification de l'amplicon obtenu par la PCR à partir de l'intensité de la fluorescence.

3. Procédé selon la revendication 1, dans lequel :
chacune des régions contient 5 à 100 des cibles auxquelles sont respectivement affectées une pluralité de sondes incluant des matériaux fluorescents présentant des longueurs d'onde de fluorescence identiques les unes aux autres ; et
lors de la quantification de l'amplicon, pour chacune des régions, l'amplicon est quantifié en mesurant collectivement une intensité de fluorescence à partir de la pluralité de sondes, indépendamment d'une différence entre les cibles.

4. Procédé selon l'une quelconque des revendications 1 à 3, dans lequel :
l'échantillon d'ADN contient un ensemble d'ADN génomique et d'ADN mitochondrial, tous deux dans la cellule unique ;
le système réactionnel PCR contient une première sonde incluant un matériau fluorescent présentant une première longueur d'onde de fluorescence et une seconde sonde incluant un matériau fluorescent présentant une seconde longueur d'onde de fluorescence, la première sonde étant affectée à une région sur l'ADN génomique, la seconde sonde étant affectée à l'ADN mitochondrial ;
la région contient la cible ou la pluralité de cibles ;
lors de l'amplification par la PCR, dans chaun de la pluralité de compartiments de réaction, la cible ou la pluralité de cibles contenues dans l'ensemble d'ADN génomique et une cible contenue dans l'ADN mitochondrial sont amplifiées par la PCR ;
lors de la quantification de l'amplicon, dans chaun de la pluralité de compartiments de réaction, l'amplicon de l'ADN génomique et l'amplicon de l'ADN mitochondrial sont quantifiés en un cycle pendant lequel l'amplification de la cible contenue dans l'ADN mitochondrial atteint un plateau et l'amplification de la cible ou de la pluralité de cibles contenues dans l'ADN génomique atteint la phase d'amplification exponentielle ; et
lors de la quantification de l'amplicon, en mesurant une intensité de fluorescence de la première longueur d'onde de fluorescence et de la seconde longueur d'onde de fluorescence à partir de chaun de la pluralité de compartiments de réaction, le compartiment de réaction dans lequel la cible ou la pluralité de cibles contenues dans l'ADN génomique et la cible contenue dans l'ADN mitochondrial sont amplifiées est différencié du compartiment de réaction dans lequel seul un acide nucléique acellulaire contaminant est amplifié.

5. Procédé selon l'une quelconque des revendications 1 à 4, comprenant en outre, la génération du compartiment de réaction en lysant la cellule unique dans un compartiment contenant la cellule unique, un réactif de lyse cellulaire et un prémélange PCR.

6. Procédé selon l'une quelconque des revendications 1 à 5, comprenant en outre :
la lyse de la cellule unique dans un compartiment contenant la cellule unique ; et
la génération du compartiment de réaction en combinant le compartiment contenant la cellule unique lysée avec un compartiment contenant un prémélange PCR.

7. Procédé selon l'une quelconque des revendications 1 à 6, comprenant en outre :
le mélange en masse d'une population de noyaux cellulaires et d'un prémélange PCR ; et
la génération d'une pluralité des compartiments de réaction en séparant les noyaux cellulaires, dans la population des noyaux cellulaires combinée au prémélange PCR, les uns des autres.

8. Procédé selon l'une quelconque des revendications 1 à 7, dans lequel le compartiment de réaction est une gouttelette de réaction qui est une gouttelette contenant l'échantillon d'ADN et le système réactionnel PCR.

9. Procédé selon l'une quelconque des revendications 1 à 8, dans lequel :
lors de la quantification du nombre de copies de l'acide nucléique spécifique, le nombre de copies de l'acide nucléique spécifique est quantifié en utilisant une valeur seuil en tant qu'étalon externe ; et
la valeur seuil est fixée au préalable sur la base d'un résultat de la quantification de l'amplicon obtenu par la PCR pour une cellule dans laquelle le nombre de copies de l'acide nucléique spécifique est connu.

10. Procédé selon la revendication 9, dans lequel avant que le résultat de la quantification de l'amplicon obtenu par la PCR ne soit utilisé pour fixer la valeur seuil, le résultat est corrigé en utilisant un résultat d'une quantification pour un cluster négatif.

11. Procédé selon l'une quelconque des revendications 1 à 10, dans lequel :
l'échantillon d'ADN contient un ensemble d'ADN génomique dans la cellule unique ;
et
le procédé comprend en outre la détection, à partir d'un résultat de la quantification de l'amplicon, de la présence de la cellule unique comportant au moins un élément sélectionné dans le groupe consistant en une aneuploïdie sur toute la longueur d'un chromosome, une aneuploïdie partielle d'un chromosome, une amplification génique et une délétion génique.

12. Procédé selon la revendication 11, dans lequel la cellule unique contient l'ADN génomique du moins comportant une mutation chromosomique sélectionnée parmi les suivantes :
- aneuploïdie du chromosome 21,
- aneuploïdie du chromosome 18,
- aneuploïdie du chromosome 13,
- aneuploïdie du chromosome Y,
- aneuploïdie du chromosome X,
- délétion de la région 22q11.2 sur un bras long du chromosome 22,
- délétion de la région 5q sur un bras court du chromosome 5,
- délétion de la région 15q11-q 13 sur un bras long du chromosome 15,
- amplification d'un bras long du chromosome 1,
- délétion d'un bras court du chromosome 17,
- délétion d'un bras long du chromosome 13,
- délétion d'un bras long du chromosome 4,
- délétion d'un bras long du chromosome 5,
- délétion d'un bras long du chromosome 7,
- amplification du chromosome 8,
- délétion du chromosome 11,
- aneuploïdie du chromosome 12,
- délétion d'un bras long du chromosome 20,
- délétion d'un bras long du chromosome 19,
- délétion du chromosome 1,
- délétion d'un bras long du chromosome 18,
- délétion d'un bras court du chromosome 8,
- délétion du chromosome 4,
- amplification d'un bras long du chromosome 8,
- délétion d'un bras long du chromosome 16,
- amplification d'un bras court du chromosome 5,
- amplification d'un bras long du chromosome 3,
- délétion d'un bras court du chromosome 3,
- délétion d'un bras court du chromosome 9,
- amplification génique du gène MYCN,
- amplification génique du gène HER2, et
- amplification génique du gène MET.

13. Procédé selon la revendication 11, comprenant en outre :
la génération de données qui incluent des informations indiquant si la présence de la cellule unique est détectée ou non, dans lequel
la population cellulaire est isolée du liquide amniotique ou du sang maternel de manière à contenir une cellule fœtale ; et
les données sont fournies en vue d'un diagnostic de trisomie 13, trisomie 18, trisomie 21, syndrome de Turner, syndrome du triple X, syndrome XYY, syndrome de Klinefelter, syndrome de Di George, syndrome d'Angelman, syndrome de Prader-Williou syndrome du cri du chat.

14. Procédé selon la revendication 11, comprenant en outre :
la génération de données qui incluent des informations indiquant si la présence de la cellule unique est détectée ou non, dans lequel
la population cellulaire est isolée d'un patient, et
les données sont fournies en vue d'un diagnostic de syndrome myélodysplasique, myélome multiple, éosinophilie idiopathique, leucémie éosinophilique chronique, leucémie aiguë non lymphocytaire, néoplasme myéloprolifératif, leucémie lymphoïde chronique, leucémie myéloïde aiguë, tumeur cérébrale, neuroblastome, cancer du côlon, cancer du sein, cancer de l'ovaire, cancer du col de l'utérus, cancer de l'endomètre, cancer du poumon à petites cellules, cancer du poumon non à petites cellules, cancer de la vessie, cancer du rein, cancer du foie, cancer du pancréas, cancer de l'œsophage, cancer de la thyroïde ou cancer de la tête et du cou.
